# EUROPEAN PATENT SPECIFICATION

(11) **EP 3 709 966 B1**
(45) Date of publication and mention of the grant of the patent: **11.12.2024**
(21) Application number: 18800967.4
(22) Date of filing: 15.11.2018
(51) Int. Cl.: A61K 8/81, A61Q 17/04

(54) **COMPOSITIONS COMPRISING AT LEAST ONE ACRYLIC POLYMER AND AT LEAST ONE INSOLUBLE ORGANIC SCREENING AGENT**
ZUSAMMENSETZUNGEN MIT MINDESTENS EINEM ACRYLPOLYMER UND MINDESTENS EINEM UNLÖSLICHEN ORGANISCHEN SCHUTZMITTEL
COMPOSITIONS COMPRENANT AU MOINS UN POLYMÈRE ACRYLIQUE ET AU MOINS UN FILTRE UV ORGANIQUE INSOLUBLE

(30) Priority: 15.11.2017 FR 1760732
(43) Date of publication of application: 23.09.2020
(73) Proprietor: L'OREAL, 75008 Paris (FR)
(72) Inventor: PILLOT, Aurélie, 94152 Chevilly La Rue (FR); FAGEON, Laure, 94152 Chevilly La Rue (FR); BATISTA,Alexandra, 94152 Chevilly La Rue (FR); ROUDOT, Angélina, 94152 Chevilly La Rue (FR); CANDAU, Didier, 94152 Chevilly La Rue (FR)
(74) Representative: L'Oreal
(86) International application number: PCT/EP2018/081475
(87) International publication number: WO 2019/096960

(56) References cited:
- EP-A1- 3 235 839
- US-A- 4 128 635
- US-A1- 2007 264 204

## Description

A subject of the present invention is a composition, in particular a cosmetic composition, comprising at least one photoprotective system capable of screening out UV rays containing at least one insoluble organic UV-screening agent and at least one particular acrylic polymer.

It is known that radiation with wavelengths of between 280 nm and 400 nm makes possible tanning of the human epidermis and that radiation with wavelengths of between 280 and 320 nm, known under the name of UV-B rays, harms the development of a natural tan. Exposure is also liable to induce impairment of the biomechanical properties of the epidermis, which is reflected by the appearance of wrinkles, leading to premature ageing of the skin.

It is also known that UV-A rays with wavelengths of between 320 and 400 nm penetrate more deeply into the skin than UV-B rays. UV-A rays cause immediate and persistent browning of the skin. Daily exposure to UVA rays, even of short duration, under normal conditions can result in damage to the collagen fibres and the elastin, which is reflected by a modification to the microrelief of the skin, the appearance of wrinkles and uneven pigmentation (liver spots, heterogeneity of the complexion).

Protection against UVA and UVB radiation is therefore necessary. An effective photoprotective product must protect against both UVA and UVB radiation.

Many cosmetic compositions intended for the photoprotection (UV-A and/or UV-B) of the skin have been provided to date.

These photoprotective compositions are quite often in the form of an emulsion, of oil-in-water type (i.e. a cosmetically acceptable support constituted of an aqueous dispersing continuous phase and of a fatty dispersed discontinuous phase), or of water-in-oil type (aqueous phase dispersed in a continuous fatty phase), which contains, in varying concentrations, one or more standard lipophilic organic screening agents and/or metal oxide mineral nanopigments capable of selectively absorbing the harmful UV radiation, these screening agents (and the amounts thereof) being selected as a function of the desired sun protection factor, the sun protection factor (SPF) being expressed mathematically as the ratio of the dose of UV radiation required to reach the erythema-forming threshold with the UV-screening agent to the dose of the UV radiation required to reach the erythema-forming threshold without the UV-screening agent. In such emulsions, the hydrophilic screening agents are present in the aqueous phase and the lipophilic screening agents are present in the fatty phase.

Oil-in-water emulsions are, in general, more appreciated by consumers than water-in-oil emulsions, especially on account of their pleasant feel (similar to water) and of their presentation in the form of milk or non-greasy cream; however, they also lose their UV-protecting efficacy more easily once they come into contact with water; specifically, hydrophilic screening agents have a tendency to disappear with water, on bathing in the sea or in a swimming pool, in the shower or when practising water sports; thus, the antisun compositions that contain them, alone or combined with lipophilic screening agents, no longer afford the desired initial protection once the substrate (skin or hair) onto which they have been applied comes into contact with water.

The most commonly used UV-screening agents are organic and soluble in oils or in aqueous media; they generally have in their structure a chromophore group linked to a solubilizing group which is generally a fatty chain in the case of liposoluble UV-screening agents or else a carboxylic or sulfonic acid group in the case of water-soluble UV-screening agents.

Most UV-screening agents have the drawback that they have to be solubilized in large amounts of oil, which often results in formulation difficulties and also in sensory drawbacks in the formula, such as a greasy effect on application.

Insoluble organic screening agents therefore appear to be particularly advantageous since they can be dispersed in water and introduced directly into the aqueous phase. Those mentioned in patent US 5 869 030 are in particular known. Among these screening agents, mention may most particularly be made of Methylenebis(benzotriazolyl)tetramethylbutylphenol sold in solid form under the trade name Mixxim BB/100^{®} by Fairmount Chemical, or in micronized form as an aqueous dispersion under the trade name Tinosorb M^{®} by the company BASF. This UV-screening agent absorbs both UVA radiation and UVB radiation.

Unfortunately, the incorporation of insoluble UV-screen agents into conventional formulations, such as oil/water or water/oil emulsions, is often difficult to carry out. This is because these insoluble screening agents have a tendency to agglomerate and/or to sediment. The compositions are not stable over time. Furthermore, the cosmetic properties are not optimal; in particular, these screening agents make products feel very dragging on application and very coarse after application.

Acrylate polymers are known in the field of cosmetics, in particular in the documents US2007/264204, EP3235839, US4128635.

The applicant has discovered, surprisingly and unexpectedly, that particular compositions containing at least one insoluble UV-screening agent and at least one particular acrylic polymer make it possible to obtain stable screening compositions which not only have cosmetic performance levels that are comparable to those generally obtained with a conventional composition in oil/water emulsion form, but are also less dragging and less coarse.

These discoveries form the basis of the present invention.

A subject of the present invention is a composition, in particular a cosmetic composition, comprising at least one insoluble organic UV-screening agent and at least one acrylic polymer defined below.

A composition in accordance with the invention may be a non-therapeutic cosmetic composition, and thus comprises a physiologically acceptable medium.

The term "human keratin materials" is intended to mean the skin (of the body, face and around the eyes), hair, eyelashes, eyebrows, body hair, nails, lips or mucous membranes.

The term "physiologically acceptable" is intended to mean compatible with the skin and/or its appendages, which has a pleasant colour, odour and feel, which does not cause any unacceptable discomfort and which is perfectly compatible with topical administration on the skin and skin appendages, and compatible with all keratin materials.

The term "insoluble UV-screening agent" is intended to mean any organic cosmetic compound for screening out UV radiation which has a solubility in water of less than 0.5% by weight and a solubility of less than 0.5% by weight in the majority of organic solvents such as paraffin oil, fatty alcohol benzoates and fatty acid triglycerides, for example Miglyol 812^{®} sold by the company Dynamit Nobel. This solubility, determined at 70°C, is defined as the amount of product in solution in the solvent at equilibrium with an excess of solid in suspension after returning to ambient temperature. It may be readily evaluated in the laboratory.

The term organic "UV-screening agent" is intended to mean any organic chemical molecule capable of absorbing and/or of physically blocking (in particular reflection or diffraction) UV radiation in the wavelength range between 280 and 400 nm.

The term "inorganic UV-screening agent" is intended to mean any non-organic chemical molecule capable of absorbing and/or of physically blocking (in particular reflection or diffraction) UV radiation in the wavelength range between 280 and 400 nm.

The term "between X and Y" is intended to mean the range of values also including the limits X and Y

According to the invention, the term "preventing" or "prevention" is intended to mean reducing the risk of occurrence or slowing down the occurrence of a given phenomenon, namely, according to the present invention, the signs of ageing of a keratin material

In the following text, the expression *"at least one"* is equivalent to "one or more" and, unless otherwise indicated, the limits of a range of values are included in that range.

Other characteristics, aspects and advantages of the present invention will emerge on reading the detailed description that follows.

### ACRYLIC POLYMER

The composition in accordance with the invention comprises at least one polymer comprising monomer units of formulae (A) and (B): 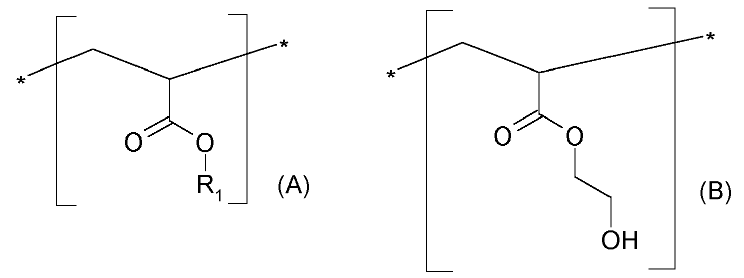 in which:
R₁, independently of one another, is chosen from alkyl or alkylene radicals;
   and
at least 60% by weight of the R₁ groups are behenyl radicals, the percentage by weight relating to the sum of all the R₁ groups present in the polymer;
   and
the weight ratio of the sum of all the hydroxyethyl acrylate units to the sum of all the acrylate units bearing the R₁ group ranges from 1: 30 to 1: 1 ,
and the sum of the total of units A and B is at least 95% by weight of the total weight of the polymer.

Preferably, R₁ is constituted of alkyl radicals, preferably of C₁₆-C₂₂ alkyl radicals, and more preferentially of behenyl (C₂₂) radicals.

Preferably, at least 70% by weight of the R₁ groups are behenyl radicals, preferentially at least 80% by weight, more preferentially at least 90% by weight. According to a preferred embodiment, all the groups R₁ are behenyl radicals.

Preferably, said weight ratio ranges from 1:15 to 1:1, preferentially ranges from 1:10 to 1:4.

Advantageously, the polymer units present in the polymer a) are constituted of the units (A) and (B) previously described.

The polymer a) has a number-average molecular weight Mn ranging from 2000 to 9000 g/mol, preferably ranging from 5000 to 9000 g/mol. The number-average molecular weight can be measured by the gel permeation chromatography method, for example according to the method described in the example hereinbelow. Preferably, the polymer a) has a melting point ranging from 60°C to 69°C, and preferentially ranging from 63°C to 67°C. The melting point is measured by differential scanning calorimetry (DSC), for example according to the method described in the example hereinbelow.

The polymer a) used according to the invention can be prepared by polymerization of a monomer of formula
CH₂=CH-COO-R₁ , R₁ having the meaning previously described, and of 2-hydroxyethyl acrylate.

The polymerization may be performed according to known methods, such as solution polymerization or emulsion polymerization.

The polymerization is, for example, described in US 2007/0264204.

The polymer(s) b) in accordance with the invention are present in the compositions in concentrations ranging from 0.1% to 10%, even more preferentially from 0.2% to 5% by weight and even more particularly from 0.5% to 4% by weight.

### INSOLUBLE ORGANIC UV SCREENING AGENTS

The insoluble organic UV-screening agents according to the invention preferably have a mean particle size which ranges from 0.01 to 5 µm and more preferentially from 0.01 to 2 µm and more particularly from 0.020 to 2 µm.

The mean particle diameter is measured using a particle size distribution analyzer of the Culter N4 PLUS type manufactured by Beckman Coulter Inc.

The insoluble organic screening agents according to the invention can be brought to the desired particulate form by any ad hoc means, such as in particular dry milling or milling in a solvent medium, sieving, atomization, micronization or pulverization.

The insoluble organic screening agents according to the invention in micronized form can in particular be obtained by means of a process of milling an insoluble organic UV-screening agent in the form of particles of coarse size in the presence of an appropriate surfactant making it possible to improve the dispersion of the resulting particles in the cosmetic formulations.

An example of a process for micronization of insoluble organic screening agents is described in applications GB-A-2 303 549 and EP-A-893119. The milling apparatus used according to these documents may be a jet, ball, vibration or hammer mill and preferably a high speed stirring mill or an impact mill and more particularly a rotating ball mill, a vibrating mill, a tube mill or a rod mill.

According to this particular process, use is made, as surfactants for milling said screening agents, of alkylpolyglucosides having the structure CₙH₂ₙ₊₁ O(C₆H₁₀O₅)ₓH in which n is an integer from 8 to 16 and x is the mean degree of polymerization of the unit (C₆H₁₀O₅) and ranges from 1.4 to 1.6. They may be chosen from C₁-C₁₂ esters of a compound having the structure CₙH₂ₙ₊₁ O(C₆H₁₀O₅)ₓH and more particularly an ester obtained by reacting a C₁-C₁₂ carboxylic acid, such as formic, acetic, propionic, butyric, sulfosuccinic, citric or tartaric acid, with one or more free OH functions on the glucoside unit (C₆H₁₀O₅). Decylglucoside may in particular be mentioned as alkyl polyglucoside.

Said surfactants are generally used at a concentration ranging from 1% to 50% by weight and more preferentially from 5% to 40% by weight, relative to the insoluble screening agent in its micronized form.

The insoluble organic UV-screening agents in accordance with the invention may be chosen in particular from organic UV-screening agents of the oxalanilide type, of the triazine type, of the benzotriazole type; of the vinylamide type; of the cinnamide type; of the type comprising one or more groups which are benzazole and/or benzofuran, benzothiophene or of the indole type; of the aryl vinylene ketone type; of the phenylene bis-benzoxazinone derivative type; of the amide, sulfonamide or acrylonitrile carbamate derivative type, or mixtures thereof.

For the purpose for which it is used in the present invention, the term "benzazole" encompasses at the same time benzothiazoles, benzoxazoles and benzimidazoles.

### A/ Oxalanides

Among the UV-screening agents of the oxalanilide type in accordance with the invention, mention may be made of those corresponding to the structure: 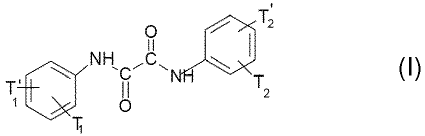 in which T₁, T'₁, T₂ and T'₂ denote, identically or differently, a C₁ to C₈ alkyl radical or a C₁ to C₈ alkoxy radical. These compounds are described in patent application WO 95/22959.

By way of example, mention may be made of the commercial products Tinuvin 3150 and Tinuvin 3120 sold by the company BASF and respectively having the structure:

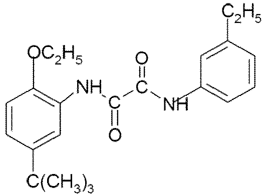

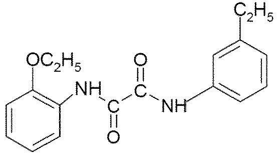

### BZ Triazines

Among the insoluble UV-screening agents of the triazine type in accordance with the invention, mention may also be made of those corresponding to formula (II) below: 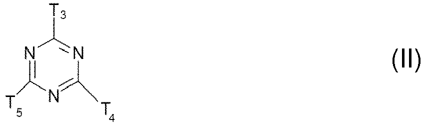 in which T₃, T₄ and T₅, independently, are phenyl, phenoxy or pyrrolo, in which the phenyl, phenoxy and pyrrolo are unsubstituted or substituted with one, two or three substituents chosen from OH, C₁-C₁₈ alkyl or C₁-C₁₈ alkoxy, C₁-C₁₈ carboxyalkyl, C₅-C₈ cycloalkyl, a methylbenzylidenecamphor group, a -(CH=CH)ₙ(CO)-OT₆ group, with T₆ being either C₁-C₁₈ alkyl or cinnamyl.

These compounds are described in WO 97/03642, GB 2286774, EP-743309, WO 98/22447 and GB 2319523.

Among the UV-screening agents of the triazine type in accordance with the invention, mention may also be made of insoluble derivatives of s-triazine bearing benzalmalonate and/or phenyl cyanoacrylate groups, such as those described in application EP-A-0790243 (which is an integral part of the content of the description).

Among these insoluble UV-screening agents of the triazine type, mention will more particularly be made of the following compounds:
- 2,4,6-tris(diethyl 4'-aminobenzalmalonate)-s-triazine,
- 2,4,6-tris(diisopropyl 4'-aminobenzalmalonate)-s-triazine,
- 2,4,6-tris(dimethyl 4'-aminobenzalmalonate)-s-triazine,
- 2,4,6-tris(ethyl α-cyano-4-aminocinnamate)-s-triazine.

Among the UV-screening agents of the triazine type in accordance with the invention, mention may also be made of insoluble derivatives of s-triazine bearing benzotriazole and/or benzothiazole groups, such as those described in application WO 98/25922 (which is an integral part of the content of the description).

Among these compounds, mention may more particularly be made of:
- 2,4,6-tris[(3'-benzotriazol-2-yl-2'-hydroxy-5'-methyl)phenylamino]-s-triazine,
- 2,4,6-tris[(3'-benzotriazol-2-yl-2'-hydroxy-5'-tert-octyl)phenylamino]-s-triazine.

Mention may also be made of the symmetrical triazines substituted with naphthalenyl groups or polyphenyl groups described in patent US 6 225 467, patent application WO 2004/085412 (see compounds 6 and 9) or the document "Symmetrical Triazine Derivatives" IP.COM Journal, IP.COM INC West Henrietta, NY, US (20 September 2004), in particular 2,4,6-tris(biphenyl)triazines and 2,4,6-tris(terphenyl)triazine which is also mentioned in patent applications WO 06/035 000, WO 06/034 982, WO 06/034 991, WO 06/035 007, WO 2006/034 992 and WO 2006/034 985.

Mention may also be made of 5,6,5',6'-tetraphenyl-3,3'-(1,4-phenylene)bis[1,2,4-triazine] (Phenylene bis-diphenyltriazine).

### C/ Benzotriazoles

Among the insoluble organic UV-screening agents of the benzotriazole type in accordance with the invention, mention may be made of those of formula (III) below, as described in application WO 95/22959 (which forms an integral part of the content of the description): 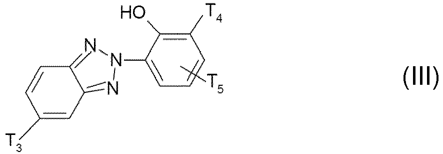 in which T₇ denotes a hydrogen atom or a C₁ to C₁₈ alkyl radical; and T₈ and T₉, which may be identical or different, denote a C₁ to C₁₈ alkyl radical optionally substituted with a phenyl.

As examples of compounds of formula (III), mention may be made of the commercial products Tinuvin 328, 320, 234 and 350 from the company BASF, having the structure below:

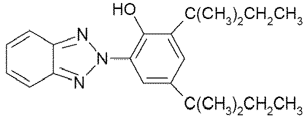

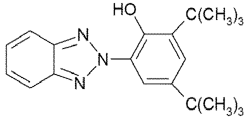

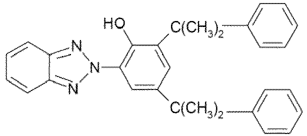

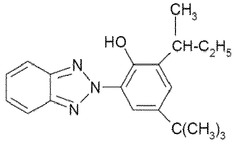

Among the insoluble organic UV-screening agents of the benzotriazole type in accordance with the invention, mention may be made of the compounds as described in patents US 5 687 521, US 5 373 037 and US 5 362 881 and in particular [2,4'-dihydroxy-3-(2H-benzotriazol-2-yl)-5-(1,1,3,3-tetramethylbutyl)-2'-n-octoxy-5'-benzoyl]diphenylmethane sold under the name Mixxim PB30^{®} by the company Fairmount Chemical, of structure:

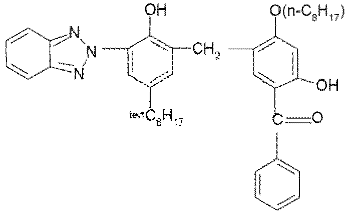

Among the insoluble organic UV-screening agents of the benzotriazole type in accordance with the invention, mention may be made of the methylenebis(hydroxyphenylbenzotriazole) derivatives having the structure below: 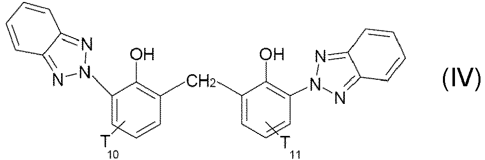 in which the radicals T₁₀ and T₁₁, which may be identical or different, denote a C₁ to C₁₈ alkyl radical which may be substituted with one or more radicals chosen from C₁-C₄ alkyl, C₅-C₁₂ cycloalkyl or an aryl residue. These compounds are known per se and described in applications 5 US 5237 071, US 5 166 355, GB-A-2 303 549, DE 197 26 184 and EP-A-893 119 (which are an integral part of the description).

In formula (I) defined above: the C₁-C₁₈ alkyl groups may be linear or branched and are, for example, methyl, ethyl, n-propyl, isopropyl, n-butyl, isobutyl, tert-butyl, tert-octyl, n-amyl, n-hexyl, n-heptyl, n-octyl, isooctyl, n-nonyl, n-decyl, n-undecyl, n-dodecyl, tetradecyl, hexydecyl or octadecyl; the C₅-C₁₂ cycloalkyl groups are, for example, cyclopentyl, cyclohexyl or cyclooctyl; the aryl groups are, for example, phenyl or benzyl.

Among the compounds of formula (IV), mention may be made of those having the structure below:

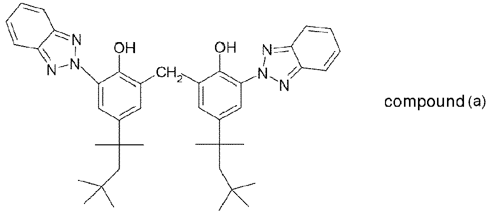

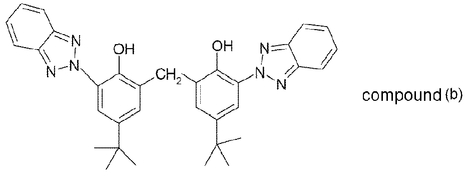

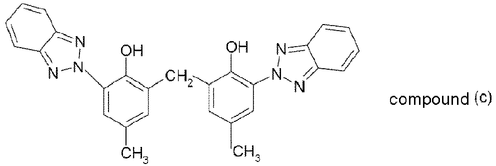

Compound (a) of nomenclature 2,2'-methylenebis[6-(2H-benzotriazol-2-yl)-4-(1,1,3,3-tetramethylbutyl)phenol] is sold especially under the trade name Mixxim BB/200^{®} by the company Fairmount Chemical.

Compound (c) of nomenclature 2,2'-methylenebis[6-(2H-benzotriazol-2-yl)-4-(methyl)phenol] is sold in particular in solid form under the trade name Mixxim BB/200^{®} by the company Fairmount Chemical.

### DZ Vinyl amides

Among the insoluble organic screening agents of the vinylamide type, mention may be made for example of the compounds having the formulae below which are described in application WO 95/22959 (which is an integral part of the content of the description):

T₁₂-(Y)r-C(=O)-C(T₁₃)=C(T₁₄)-N(T₁₅)(T₁₆) (V)

in which T₁₂ is a C₁ to C₁₈, preferably C₁ to C₅, alkyl radical or a phenyl group which is optionally substituted with one, two or three radicals chosen from OH, C₁ to C₁₈ alkyl, C₁ to C₈ alkoxy, or a -C(=O)-OT₁₇ group where T₁₇ is a C₁ to C₁₈ alkyl; T₁₃, T₁₄, T₁₅ and T₁₆, which may be identical or different, denote a C₁ to C₁₈, preferably C₁ to C₅, alkyl radical or a hydrogen atom; Y is N or O and r is 0 or 1.

Among these compounds, mention will more particularly be made of:
- 4-octylamino-3-penten-2-one;
- ethyl 3-octylamino-2-butenoate;
- 3-octylamino-1-phenyl-2-buten-1-one;
- 3-dodecylamino-1-phenyl-2-buten-1-one.

### EZ Cinnamamides

Among the insoluble organic screening agents of the cinnamamide type in accordance with the invention, mention may also be made of the compounds as described in application WO 95/22959 (which forms an integral part of the content of the description) and which correspond to the structure below: 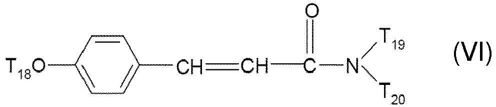 in which OT₁₈ is a hydroxyl or C₁ to C₄ alkoxy radical, preferably methoxy or ethoxy; T₁₉ is hydrogen or C₁ to C₄ alkyl, preferably methyl or ethyl; T₂₀ is a -(CONH)ₛ-phenyl group where s is 0 or 1 and the phenyl group may be substituted with one, two or three groups chosen from OH, C₁ to C₁₈ alkyl, C₁ to C₈ alkoxy, or a -C(=O)-OT₂₁ group where T₂₁ is a C₁ to C₁₈ alkyl and more preferentially T₂₁ is a phenyl, 4-methoxyphenyl or phenylaminocarbonyl group.

Mention may also be made of cinnamamide dimers such as those described in patent US 5 888 481, for instance the compound having the structure:

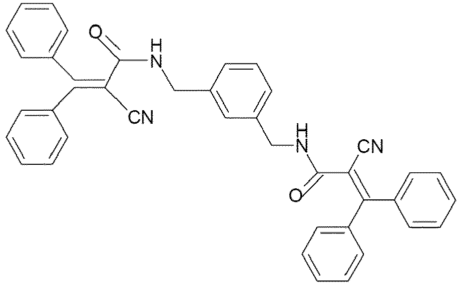

### FZ Benzazoles

Among the insoluble organic screening agents of the benzazole type, mention may be made of those corresponding to one of the formulae below: 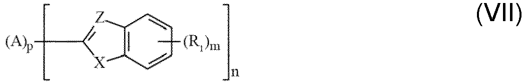 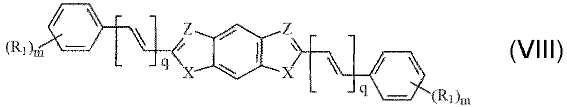 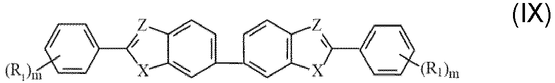
in which each of the symbols X independently represents an oxygen or sulfur atom or a group NR₂, each of the symbols Z independently represents a nitrogen atom or a CH group,
each of the symbols R₁ independently represents an OH group, a halogen atom, a linear or branched C₁-C₈ alkyl group, optionally containing a silicon atom, or a linear or branched C₁-C₈ alkoxy group,
each of the numbers m is independently 0, 1 or 2,
n represents an integer between 1 and 4 inclusive,
p is equal to 0 or 1,
each of the numbers q is independently equal to 0 or 1,
each of the symbols R2 independently represents a hydrogen atom, or a benzyl or linear or branched C₁-C₈ alkyl group, optionally containing a silicon atom,

A represents a radical of valency n chosen from those of formulae: 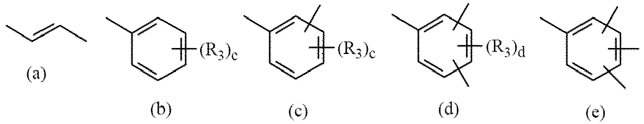 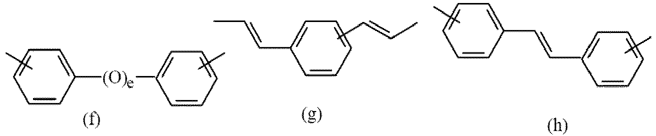 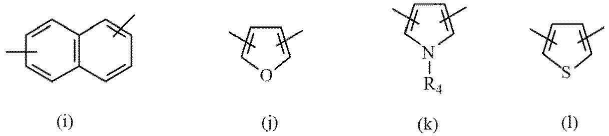 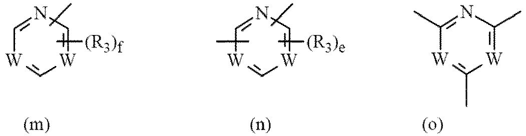 in which each of the symbols R₃ independently represents a halogen atom or a linear or branched C₁-C₄ alkyl or alkoxy group or a hydroxyl group, and R₄ represents a hydrogen atom or a linear or branched C₁-C₄ alkyl group, c = 0 - 4, d = 0 - 3, e = 0 or 1, and f = 0 - 2.

These compounds are especially described in patents DE 676 103 and CH 350 763, patent US 5 501 850, patent US 5 961 960, patent application EP0669323, patent US 5 518 713, patent US 2 463 264, the article J. Am. Chem. Soc., 79, 5706 - 5708, 1957, the article J. Am. Chem. Soc., 82, 609 - 5 611, 1960, patent application EP0921126, and patent application EP712855.

As examples of preferred compounds of formula (VII) of the 2-arylbenzazole family, mention may be made of 2-benzoxazol-2-yl-4-methylphenol, 2-(1H-benzimidazol-2-yl)-4-methoxyphenol or 2-benzothiazol-2-ylphenol, these compounds possibly being prepared, for example, according to the processes described in patent CH 350 763.

As examples of preferred compounds of formula (VII) of the benzimidazolylbenzazole family, mention will be made of 2,2'-bis-benzimidazole, 5,5',6,6'-tetramethyl-2,2'-bis-benzimidazole, 5,5'-dimethyl-2,2'-bis-benzimidazole, 6-methoxy-2,2'-bis-benzimidazole, 2-(1H-benzimidazol-2-yl)benzothiazole, 2-(1H-benzimidazol-2-yl)benzoxazole and N,N'-dimethyl-2,2'-bis-benzimidazole, these compounds possibly being prepared according to the procedures described in patents US 5 961 960 and US 2 463 264.

As examples of preferred compounds of formula (VII) of the phenylenebenzazole family, mention will be made of 1,4-phenylene-bis-(2-benzoxazolyl), 1,4-phenylene-bis-(2-benzimidazolyl), 1,3-phenylene-bis-(2-benzoxazolyl), 1,2-phenylene-bis-(2-benzoxazolyl), 1,2-phenylene-bis-(benzimidazolyl), 1,4-phenylene-bis-(N-2-ethylhexyl-2-benzimidazolyl) and 1,4-phenylene-bis-(N-trimethylsilylmethyl-2-benzimidazolyl), these compounds possibly being prepared according to the procedures described in patent US 2 463 264 and in the publications J. Am. Chem. Soc., 82, 609 (1960) and J. Am. Chem. Soc., 79, 5706 - 5708 (1957).

As examples of preferred compounds of formula (VII) of the benzofuranylbenzoxazole family, mention will be made of 2-(2-benzofuranyl)benzoxazole, 2-(benzofuranyl)-5-methylbenzoxazole and 2-(3-methyl-2-benzofuranyl)benzoxazole, these compounds possibly being prepared according to the procedures described in patent US 5 518 713.

As preferred compounds of formula (VIII), mention may be made, for example, of 2,6-diphenyl-1,7-dihydrobenzo[1,2-d;4,5-d']diimidazole corresponding to the formula: 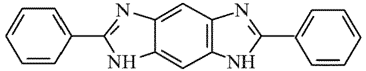 or 2,6-distyryl-1,7-dihydrobenzo[1,2-d; 4,5-d']diimidazole or else 2,6-di(p-tert-butylstyryl)-1,7-dihydrobenzo[1,2-d; 4,5-d']diimidazole, which may be prepared according to application EP 0 669 323.

As preferred compound of formula (IX), mention may be made of 5,5'-bis-[(phenyl-2)-benzimidazole] having the formula: 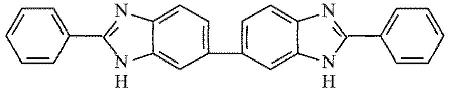 the preparation of which is described in J. Chim. Phys., 64, 1602 (1967).

Among these insoluble organic compounds which screen out UV radiation, preference is given most particularly to 2-(1H-benzimidazol-2-yl)benzoxazole, 5 ole, 6-methoxy-2,2'-bis-benzimidazole, 2-(1H-benzimidazol-2-yl)benzothiazole, 1,4-phenylenebis-(2-benzoxazolyl), 1,4-phenylene-bis-(2-benzimidazolyl), 1,3-phenylenebis-(2-benzoxazolyl), 1,2-phenylene-bis-(2-benzoxazolyl), 1,2-phenylenebis-(2-benzimidazolyl) and 1,4-phenylene-bis-(N-trimethylsilylmethyl-2-benzimidazolyl).

### G/ Aryl vinylene ketones

Among the insoluble organic screening agents of the aryl vinylene ketone type, mention may be made of those corresponding to one of formulae (X) and (XI) below: 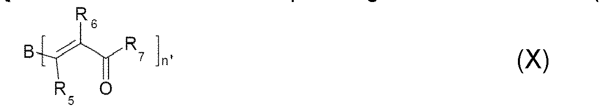 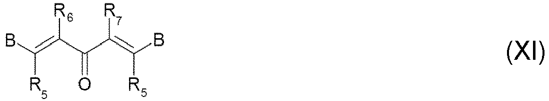 in which:
n' = 1 or 2,
B, in formula (X) when n'=1 or in formula (XI), is an aryl radical chosen from formulae (a') to (d') below, or, in formula (X) when n'=2, is a radical chosen from formulae (e') to (h') below: 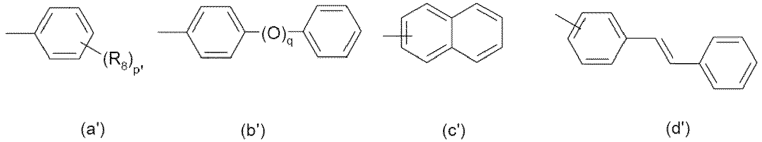 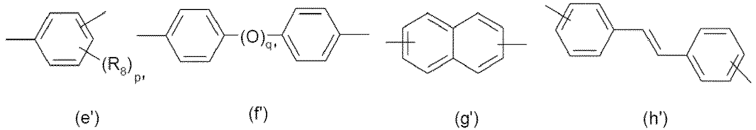 in which:
   each of the symbols R₈ independently represents an OH group, a halogen atom, a linear or branched C₁-C₆ alkyl group, optionally containing a silicon atom, a linear or branched C₁-C₆ alkoxy group, optionally containing a silicon atom, a linear or branched C₁-C₅ alkoxycarbonyl group, or a linear or branched C₁-C₆ alkylsulfonamide group, optionally containing a silicon atom or an amino acid function,
   p' represents an integer between 0 and 4 inclusive,
   q' represents 0 or 1,
   R₅ represents hydrogen or an OH group,
   R₆ represents hydrogen, a linear or branched C₁-C₆ alkyl group, optionally containing a silicon atom, a cyano group, a C₁-C₆ alkylsulfonyl group, or a phenylsulfonyl group,
   R₇ represents a linear or branched C₁-C₆ alkyl group, optionally containing a silicon atom, or a phenyl group which can form a bicycle and which is optionally substituted with one or two radicals R₄,
   or R₆ and R₇ together form a monocyclic, bicyclic or tricyclic C₂₋₁₀ hydrocarbon-based residue, optionally interrupted with one or more nitrogen, sulfur and oxygen atoms and which can contain another carbonyl, and optionally substituted with a linear or branched C₁-C₈ alkylsulfonamide group,
   and optionally containing a silicon atom or an amino acid function; provided that, when n' = 1, R₆ and R₇ do not form a camphor nucleus.

As examples of insoluble compounds of formula (X), in which n' = 1, which screen out UV radiation and which have a mean particle size of between 10 nm and 5 nm, mention may be made of the following families:
- compounds of the styryl ketone type as described in application JP 04 134 042, such as 1-(3,4-dimethoxyphenyl)-4,4-dimethylpent-1-en-3-one: 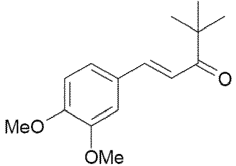
- compounds of the benzylidene cineole type such as those described in the article by E. Mariani et al, 16th IFSCC Congress, New York (1990), such as 1,3,3-trimethyl-5-(4-methoxybenzylidene)-2-oxabicyclo[2.2.2]octan-6-one: 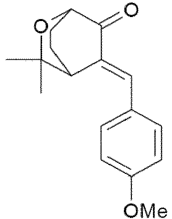
- compounds of the benzylidene chromanone type such as those described in application JP 04 134 043, for instance 3-(4-methoxybenzylidene)-2,3,4a,8a-tetrahydrochromen-4-one: 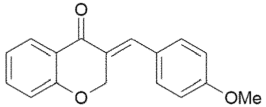
- compounds of the benzylidene thiochromanone type such as those described in application JP 04 134 043, for instance 3-(4-methoxybenzylidene)-2,3,4a,8a-tetrahydrochromen-4-thione: 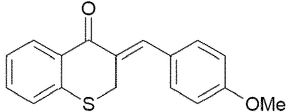
- compounds of the benzylidene quinuclidinone type such as those described in application EP 0 576 974, for instance 4-methoxybenzylidene-1-azabicyclo[2.2.2]octan-3-one: 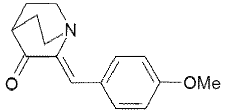
- compounds of the benzylidene cycloalcanone type such as those described in application FR 2 395 023, for instance 2-(4-methoxybenzylidene)cyclopentanone and 2-(4-methoxybenzylidene)cyclohexanone: 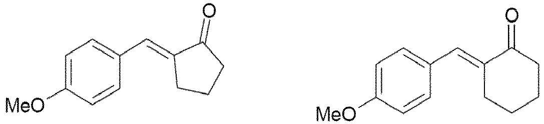
- compounds of the benzylidene hydantoin type such as those described in application JP 01 158 090, for instance 5-(3,4-dimethoxybenzylidene)imidazolidine-2,4-dione: 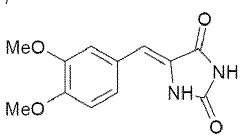
- compounds of the benzylidene indanone type such as those described in application JP 04 134 043, for instance 2-(4-methoxybenzylidene)indan-1-one: 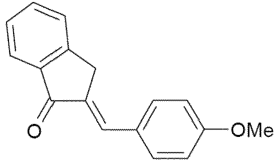
- compounds of the benzylidene tetralone type such as those described in application JP 04 134 043, for instance 2-(4-methoxybenzylidene)-3,4-dihydro-2H-naphthalen-1-one: 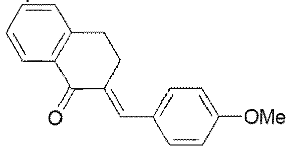
- compounds of the benzylidene furanone type such as those described in application EP 0 390 683, for instance 4-(4-methoxybenzylidene)-2,2,5,5-tetramethyldihydrofuran-3-one: 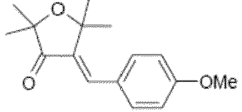
- compounds of the benzylidene benzofuranone type such as those described in application JP 04 134 041, for instance 2-benzylidenebenzofuran-3-one: 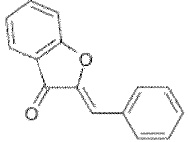
- compounds of the benzylidene indanedione type such as 2-(3,5-di(tert-butyl)-4-hydroxybenzylidene)indan-1,3-dione: 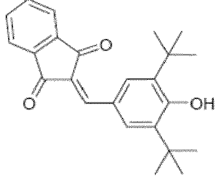
- compounds of the benzylidene benzothiofuranone type such as those described in application JP 04,134,043, for instance 2-benzylidenebenzo[b]thiophen-3-one: 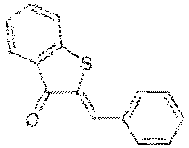
- compounds of the benzylidene barbiturate type such as 5-(4-methoxybenzylidene)-1,3-dimethylpyrimidine-2,4,6-trione: 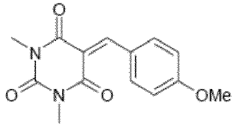
- compounds of the benzylidene pyrazolone type such as 4-(4-methoxybenzylidene)-5-methyl-2-phenyl-2,4-dihydropyrazol-3-one: 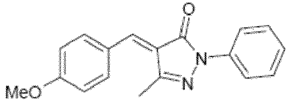
- compounds of the benzylidene imidazolone type such as 5-(4-methoxybenzylidene)-2-phenyl-3,5-dihydroimidazol-4-one: 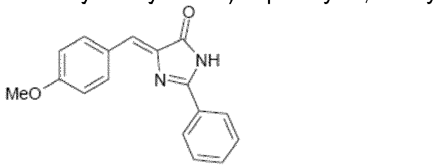
- compounds of the chalcone type such as 1-(2-hydroxy-4-methoxyphenyl)-3-phenylpropenone: 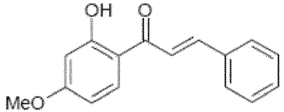
- benzylidene one compounds as described in document FR 2 506 156, for instance 3-hydroxy-1-(2-hydroxy-4-methoxyphenyl)-3-phenylpropenone:

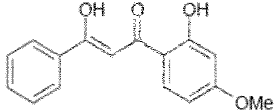

As examples of insoluble compounds of formula (X), in which n' = 2, which screen out UV radiation and which have a mean particle size of between 10 nm and 5 µm, mention may be made of the following families:
- compounds of the phenylene bis methylidene-nor-camphor type as described in document EP 0 693 471, for instance 1,4-phenylene-bis-{3-methylidenebicyclo[2.2.1]heptan-2-one}: 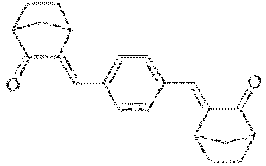
- compounds of the phenylene bis methylidene camphor type as described in document FR 2 528 420, for instance 1,4-phenylene-bis-{3-methylidene-1,7,7-trimethylbicyclo[2.2.1]heptan-2-one}: 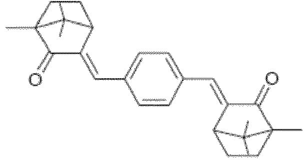 or 1,3-phenylene-bis-{3-methylidene-1,7,7-trimethylbicyclo [2.2.1]heptan-2-one}: 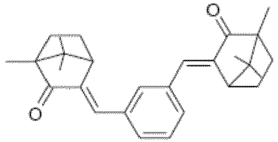
- compounds of the phenylene bis methylidene camphor sulfonamide type such as those described in document FR2 529 887, for instance ethyl or 2-ethylhexyl 1,4-phenylene-bis-3,3'-methylidenecamphor-10,10'-sulfonamide: 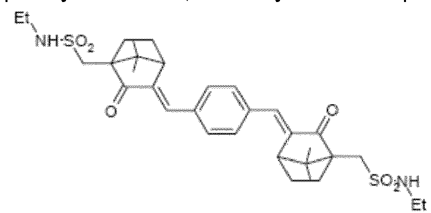 or 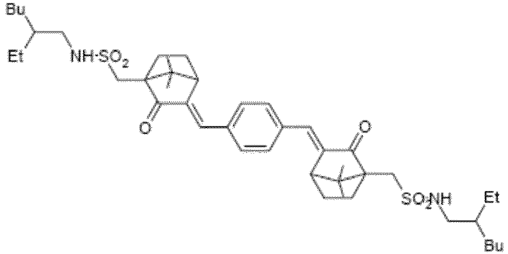
- compounds of the phenylene bis methylidene cineole type as described in the article by E. Mariani et al., 16th IFSCC Congress, New York (1990), for instance 1,4-phenylene-bis-{5-methylidene-3,3-dimethyl-2-oxabicyclo[2.2.2]octan-6-one}: 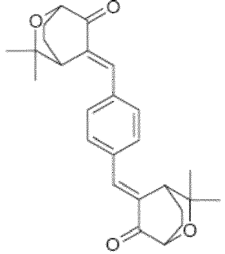
- compounds of the phenylene bis methylidene ketotricyclodecane type as described in application EP 0 694 521, for instance 1,4-phenylene-bis-(octahydro-4,7-methano-6-inden-5-one): 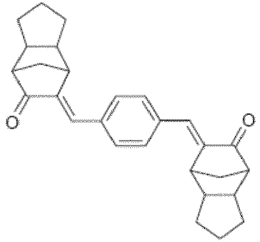
- compounds of the phenylene bis alkylene ketone type such as those described in application JP 04 134 041, for instance 1,4-phenylene-bis-(4,4-dimethyl-pent-1-en-3-one): 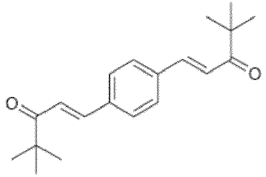
- compounds of the phenylene bis methylidene furanone type as described in application FR 2 638 354, for instance 1,4-phenylene-bis-(4-methylidene-2,2,5,5-tetramethyldihydrofuran-3-one): 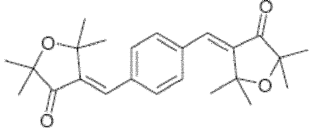
- compounds of the phenylene bis methylidene quinuclidinone type such as those described in application EP 0 714 880, for instance 1,4-phenylene-bis-{2-methylidene-1-azabicyclo[2.2.2]octan-3-one}:

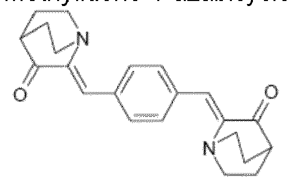

As compounds of formula (XI), mention may be made of the following families:
- compounds of the bis benzylidene cycloalcanone type such as 2,5-dibenzylidenecyclopentanone: 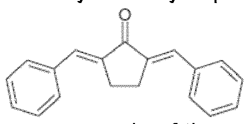
- compounds of the gamma pyrone type as described in document JP 04 290 882, for instance 2,6-bis-(3,4-dimethoxyphenyl)pyran-4-one:

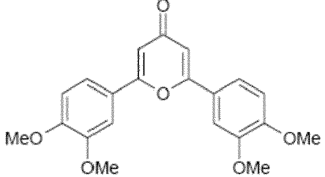

Among these insoluble organic compounds which screen out UV radiation, of the aryl vinylene ketone type, preference is given most particularly to the compounds of formula (X) in which n' = 2.

### HZ Phenylene bis-benzoxazinones

Among the insoluble organic screening agents of the phenylene bis-benzoxazinone type, mention may be made of those corresponding to formula (XII) below: 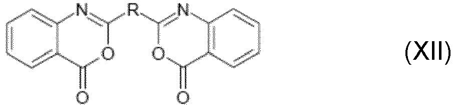 with R representing a divalent aromatic residue chosen from the formulae (e) to (h) below: 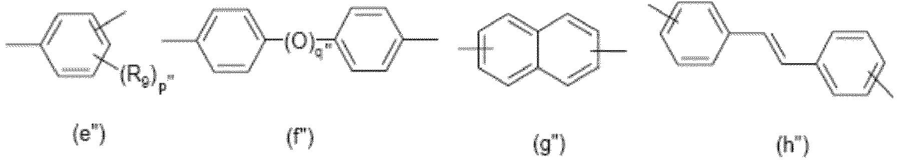 in which:
each of the symbols R₉ independently represents an OH group, a halogen atom, a linear or branched C₁-C₆ alkyl group, optionally containing a silicon atom, a linear or branched C₁-C₆ alkoxy group, optionally containing a silicon atom, a linear or branched C₁-C₅ alkoxycarbonyl group, or a linear or branched C₁-C₆ alkylsulfonamide group, optionally containing a silicon atom or an amino acid function,
p" represents an integer between 0 and 4 inclusive,
q" represents 0 or 1.

As examples of insoluble compounds of formula (XII), which screen out UV radiation and which have a mean particle size of between 10 nm and 5 µm, mention may be made of the following derivatives:
- 2,2'-p-phenylenebis(3,1-benzoxazin-4-one), commercial product Cyasorb UV-3638^{®} from the company Cytec,
- 2,2'-(4,4'-biphenylene)bis(3,1-benzoxazin-4-one),
- 2,2'-(2,6-naphthylene)bis(3,1-benzoxazin-4-one).

### I/ Acrylonitrile amide, sulfonamide or carbamate derivatives

Among the insoluble organic screening agents of the acrylonitrile amide, sulfonamide or carbamate derivative type, mention may be made of those corresponding to formula (XIII) below: 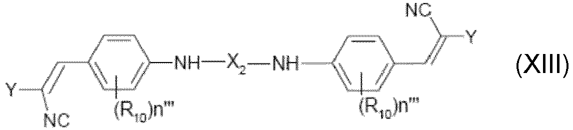 in which:
R₁₀ represents a linear or branched C₁-C₈ alkyl group,
n‴ is 0, 1 or 2,
X₂ represents a divalent radical of formula -(C=O)-R₁₁-(C=O)-, -SO₂-R₁₁-SO₂- or - (C=O)-O-Rn-O-(C=O)-,
Y represents a radical -(C=O)-R₁₂ or -SO₂R₁₃,
R₁₁ represents a single bond or a linear or branched C₁-C₃₀ alkylene or C₃-C₃₀ alkenylene divalent radical which may bear one or more hydroxyl substituents and which may contain, in the carbon-based chain, one or more heteroatoms chosen from oxygen, nitrogen and silicon atoms,
R₁₂ represents a radical -OR₁₄ or -NHR₁₄,
R₁₃ represents a linear or branched C₁-C₃₀ alkyl radical, or a phenyl nucleus which is unsubstituted or substituted with C₁-C₄ alkyl or alkoxy radicals,
R₁₄ represents a linear or branched C₁-C₃₀ alkyl or C₃-C₃₀ alkenyl radical which may bear one or more hydroxyl substituents and which may contain, in the carbon-based chain, one or more heteroatoms chosen from oxygen, nitrogen and silicon atoms.

Although, in formula (XIII) above, only the isomers in which the cyano substituent is in the cis position relative to the para-aminophenyl substituent are represented, this formula should be understood as also encompassing the corresponding *trans* isomers; for each of the two double bonds and independently, the cyano and para-aminophenyl substituents may be in the cis or *trans* configuration relative to one another.

By way of example, mention may be made of the dimer of 2-ethylhexyl 2-cyano-3-[4-(acetylamino)phenyl]acrylate of formula:

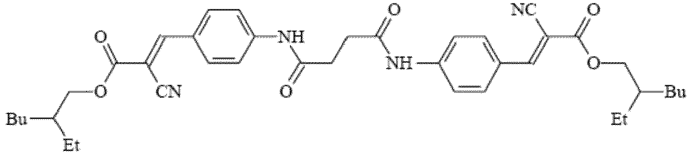

### J/ Polyvalent metals

Another particular family of insoluble organic screening agents in accordance with the invention are the salts of polyvalent metals (for example Ca²⁺, Zn²⁺, Mg²⁺, Ba²⁺, Al³⁺ or Zr⁴⁺ ) of sulfonic or carboxylic organic screening agents such as the polyvalent metal salts of sulfonated derivatives of benzylidenecamphor, such as those described in application FR-A 2 639 347; the polyvalent metal salts of sulfonated derivatives of benzimidazole, such as those described in application EP-A-893119; the polyvalent metal 5 salts of cinnamic acid derivatives, such as those described in patent application JP-87 166 517.

Mention may also be made of the metal, ammonium or substituted-ammonium complexes of UV-A and/or UV-B organic screening agents as described in patent applications WO93/10753, WO93/11095 and WO95/05150.

Among the insoluble organic UV-screening agents, mention may also be made of the compound 1,1'-(1,4-piperazinediyl)bis[1-[2-[4-(diethylamino)-2-hydroxybenzoyl]phenyl]methanone (CAS 919803-06-8) having the following structure: 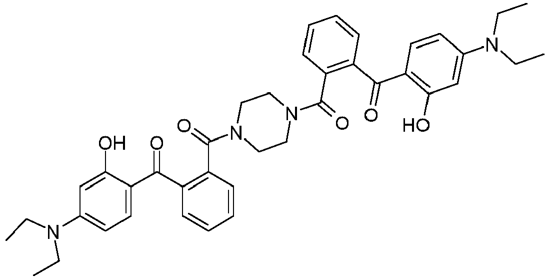 as described in application WO 2007/071 584; this compound advantageously being used in micronized form (mean size of 0.02 to 2 µm), which may be obtained, for example, according to the micronization process described in applications GB-A-2 303 549 and EP-A-893 119, and in particular in the form of an aqueous dispersion.

According to one particularly preferred form of the invention, use will be made of the insoluble organic UV-screening agents chosen from:
(i) symmetrical triazine screening agents substituted with naphthalenyl groups or polyphenyl groups described in patent US 6 225 467, application WO 2004/085 412 (see compounds 6 and 9) or the document "Symmetrical Triazine Derivatives", IP.COM IPCOM000031257 Journal, INC West Henrietta, NY, US (20 September 2004), in particular 2,4,6-tris(diphenyl)triazine and 2,4,6-tris(terphenyl)triazine, which is also mentioned in patent applications WO 06/035 000, WO 06/034 982, WO 06/034 991, WO 06/035 007, WO 2006/034 992 and WO 2006/034 985, these compounds advantageously being used in micronized form (mean particle size of 0.02 to 3 µm), which may be obtained, for example, according to the micronization process described in applications GB-A-2 303 549 and EP-A-893 119, and in particular in aqueous dispersion form;
(ii) the methylenebis(hydroxyphenylbenzotriazole) compounds of formula (IV) below: 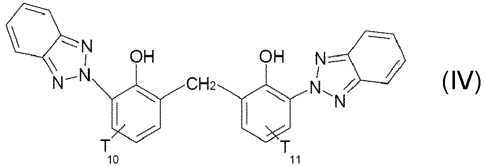 in which the radicals T₁₀ and T₁₁, which may be identical or different, denote a C₁-C₁₈ alkyl radical which may be substituted with one or more radicals chosen from C₁-C₄ alkyl, C₅-C₁₂ cycloalkyl or an aryl residue;
(iii) and mixtures thereof.

According to a particularly preferred form of the invention, the methylenebis(hydroxyphenylbenzotriazole) compounds of formula (IV) are in the form of an aqueous dispersion of particles having a mean particle size which ranges from 0.01 to 5 µm and more preferentially from 0.01 to 2 µm and more particularly from 0.020 to 2 µm with at least one surfactant of structure CₙH₂ₙ₊₁ O(C₆H₁₀O₅)ₓH in which n is an integer from 8 to 16 and x is the mean degree of polymerization of the unit (C₆H₁₀O₅) and ranges from 1.4 to 1.6 as defined previously. Said surfactant is preferably used at a concentration ranging from 1% to 50% by weight, and more preferentially from 5% to 40% by weight, relative to the benzotriazole screening agent, and the amount of benzotriazole screening agent of formula (I) in the aqueous dispersion preferably ranges from 10% to 50% by weight, and more preferentially from 30% to 50% by weight, relative to the total weight of the dispersion.

The mean particle diameter is measured using a particle size distribution analyzer of the Culter N4 PLUS^{®} type manufactured by Beckman Coulter Inc.

According to a particularly preferred form of the invention, the methylenebis(hydroxyphenylbenzotriazole) compounds of formula (IV) may be in the form of an aqueous dispersion of particles having a mean particle size which ranges from 0.02 to 2 µm and more preferentially from 0.01 to 1.5 µm and more particularly from 0.02 to 1 µm in the presence of at least one polyglycerol mono(C₈-C₂₀)alkyl ester having a degree of glycerol polymerization of at least 5, such as the aqueous dispersions described in patent application WO2009/063392.

As an example of surfactants which are mono-(C₈-C₂₀)alkyl esters of polyglycerol, mention may be made of decaglyceryl caprate, decaglyceryl laurate, decaglyceryl myristate, decaglyceryl oleate, decaglyceryl stearate, decaglyceryl isostearate, hexaglyceryl caprate, hexaglyceryl laurate, hexaglyceryl myristate, hexaglyceryl oleate, hexaglyceryl stearate, hexaglyceryl isostearate, pentaglyceryl caprate, pentaglyceryl laurate, pentaglyceryl myristate, pentaglyceryl oleate, pentaglyceryl stearate, and pentaglyceryl isostearate.

Use will more particularly be made of:
- decaglyceryl caprate such as the products sold under the following trade names: Sunsoft Q10Y^{®}, Sunsoft Q10S^{®}, Sunsoft Q12Y^{®}, Sunsoft Q12S^{®}, Sunsoft M12J^{®} by the company Taiyo Kagaku Co. Ltd., Nikkol Decaglyn 1-L by the company Nikko Chemicals Co. Ltd, Ryoto-Polyglycerylester L-10D^{®} and L-7D^{®} by the company Mitsubishi-Kagaku Co. Ltd.,
- decaglyceryl laurate such as the products sold under the following trade names: Sunsoft Q14Y^{®}, Sunsoft Q14S^{®}, Sunsoft Q12Y^{®}, Sunsoft Q12S^{®}, Sunsoft M12J^{®} by the company Taiyo Kagaku Co. Ltd., Nikkol Decaglyn 1-M^{®} by the company Nikko Chemicals Co. Ltd, Ryoto-Polyglycerylester M-10D^{®} and M-7D^{®} by the company Mitsubishi-Kagaku Co. Ltd.,
- decaglyceryl stearate such as the products sold under the following trade names: Sunsoft Q18Y^{®}, Sunsoft Q18S^{®}, Sunsoft Q12Y^{®}, Sunsoft Q12S^{®}, Sunsoft M12J^{®} by the company Taiyo Kagaku Co. Ltd., Nikkol Decaglyn 1-SV by the company Nikko Chemicals Co. Ltd, Ryoto-Polyglycerylester S-15D^{®} by the company Mitsubishi-Kagaku Co. Ltd.,
- hexaglyceryl caprate such as the products sold under the following trade names: Nikkol Hexaglyn 1-L^{®} by the company Nikko Chemicals Co. Ltd, Glysurf 6ML by the company Aoki Oil Industrial Co. Ltd., Unigly GL-106^{®} by the company Nippon Oil & Fats Co. Ltd.,
- hexaglyceryl myristate such as the products sold under the following trade names: Nikkol Hexaglyn 1-M^{®}, Nikkol Hexaglyn 1-OV^{®} by the company Nikko Chemicals Co. Ltd, Glysurf 6ML^{®} by the company Aoki Oil Industrial Co. Ltd., Unigly GL-106 by the company Nippon Oil & Fats Co. Ltd.,
- hexaglyceryl stearate such as the products sold under the following trade names: Nikkol Hexaglyn 1-M^{®}, Nikkol Hexaglyn 1-SV^{®} by the company Nikko Chemicals Co. Ltd, EmalexMSG-6K^{®} by the company Nihon-Emulsion Co. Ltd., Unigly GL-106 by the company Nippon Oil & Fats Co. Ltd.,
- hexaglyceryl isostearate such as the products sold under the following trade names: Matsumate MI-610^{®} by the company Matsumoto Fine Chemical Co. Ltd,
- pentaglyceryl caprate such as the products sold under the following trade names: Sunsoft A10E^{®}, by the company Taiyo Kagaku Co. Ltd.,
- pentaglyceryl laurate such as the products sold under the following trade names: Sunsoft A12E^{®} Sunsoft A121E^{®}, by the company Taiyo Kagaku Co. Ltd.,
- pentaglyceryl myristate such as the products sold under the following trade names: Sunsoft A14E^{®}, Sunsoft A141E^{®}, by the company Taiyo Kagaku Co. Ltd.,
- pentaglyceryl oleate such as the products sold under the following trade names: Sunsoft A17E^{®}, Sunsoft A171E^{®}, by the company Taiyo Kagaku Co. Ltd.,
- pentaglyceryl stearate such as the products sold under the following trade names: Sunsoft A18E^{®}, Sunsoft A181E^{®}, by the company Taiyo Kagaku Co. Ltd.

Among these surfactants, those having an HLB greater than or equal to 14.5, and more preferentially greater than or equal to 15, are preferably used. As examples of surfactants which are mono-(C₈-C₂₀)alkyl esters of polyglycerol having a degree of polymerization having a degree of glycerol polymerization of at least 5 and having an HLB greater than or equal to 14.5, mention may be made of decaglyceryl caprate, decaglyceryl laurate, decaglyceryl myristate, decaglyceryl oleate, decaglyceryl stearate, decaglyceryl isostearate, hexaglyceryl laurate, pentaglyceryl caprate, pentaglyceryl laurate, pentaglyceryl myristate, pentaglyceryl oleate, and pentaglyceryl stearate. As examples of surfactants which are mono-(C₈-C₂₀)alkyl esters of polyglycerol having a degree of polymerization having a degree of glycerol polymerization of at least 5 and having an HLB greater than or equal to 15, mention may be made of decaglyceryl caprate and decaglyceryl laurate.

The amount of methylenebis(hydroxyphenylbenzotriazole) compound of formula (IV) in the aqueous dispersion preferably ranges from 10% to 50% by weight, and more preferentially from 30% to 50% by weight, relative to the total weight of the dispersion.

Preferentially, the methylenebis(hydroxyphenylbenzotriazole) compound/mono-(C₈-C₂₀)alkyl ester of polyglycerol weight ratio ranges from 0.05 to 0.5, and more preferentially from 0.1 to 0.3.

In these aqueous dispersions, use will more preferentially be made, as methylenebis(hydroxyphenylbenzotriazole) compound of formula (IV), of the compound 2,2'-methylenebis[6-(2H-benzotriazol-2-yl)-4-(1,1,3,3-tetramethylbutyl)phenol] having the structure: 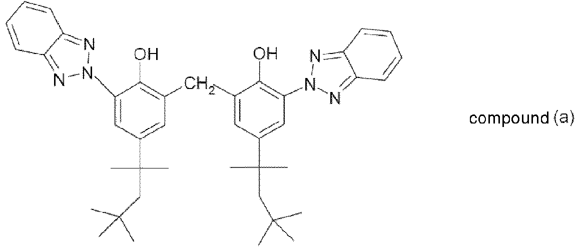 such as the commercial product sold under the name Tinosorb M^{®} by BASF which is an aqueous dispersion comprising decylglucoside, xanthan gum and propylene glycol (INCI name: Methylene Bis-Benzotriazolyl Tetramethylbutylphenol (and) Aqua (and) Decyl Glucoside (and) Propylene Glycol (and) Xanthan Gum).

The insoluble organic UV screening agent(s) of the invention are present at an active material concentration preferably ranging from 0.1% to 20% by weight approximately and more particularly from 0.5% to 10% by weight relative to the total weight of the composition.

### ADDITIONAL UV-SCREENING AGENTS

The compositions in accordance with the invention can also contain soluble organic UV-screening agents which are active in the UV-A range and/or the UV-B range, and/or inorganic screening agents. They are in particular chosen from anthranilates; cinnamic derivatives; dibenzoylmethane derivatives; salicylic derivatives, camphor derivatives; triazine derivatives such as those described in patent applications US 4 367 390, EP863145, EP517104, EP570838, EP796851, EP775698, EP878469 and EP933376; benzophenone derivatives; β,β'-diphenylacrylate derivatives, benzotriazole derivatives, benzimidazole derivatives; imadazolines; bisbenzoazolyl derivatives as described in patents EP669323 and US 2 463 264; p-aminobenzoic acid (PABA) derivatives; screening polymers and screening silicones such as those described in particular in application WO 93/04665; α-alkylstyrene-based dimers such as those described in patent application DE19855649; 4,4-diarylbutadienes such as those described in patent applications EP0967200 and DE19755649, and also mixtures thereof.

As examples of supplementary organic screening agents which are active in the UV-A range and/or UV-B range, mention may be made of those denoted above, under their INCI name:
para-Aminobenzoic acid derivatives:
   - PABA,
   - Ethyl PABA,
   - Ethyl dihydroxypropyl PABA,
   - Ethylhexyl dimethyl PABA sold in particular under the name Escalol 507 by ISP,
   - Glyceryl PABA,
   - PEG-25 PABA sold under the name Uvinul P25 by BASF,
Salicylic derivatives:
   - Homosalate, sold under the name Eusolex HMS by Rona/EM Industries,
   - Ethylhexyl salicylate sold under the name Neo Heliopan OS by Haarmann & Reimer,
   - Dipropylene glycol salicylate sold under the name Dipsal by Scher,
   - TEA salicylate sold under the name Neo Heliopan TS by Haarmann & Reimer,
Dibenzoylmethane derivatives:
   - Butylmethoxydibenzoylmethane sold in particular under the trade name Parsol 1789 by Hoffmann La Roche,
   - Isopropyldibenzoylmethane,
Cinnamic derivatives:
   - Ethylhexyl methoxycinnamate sold in particular under the trade name Parsol MCX by Hoffmann LaRoche,
   - Isopropyl methoxycinnamate,
   - Isoamyl methoxycinnamate sold under the trade name Neo Heliopan E 1000 by Haarmann & Reimer,
   - Cinoxate,
   - DEA methoxycinnamate,
   - Diisopropyl methylcinnamate,
   - Glyceryl Ethylhexanoate Dimethoxycinnamate.
β,β'-Diphenylacrylate derivatives:
   - Octocrylene sold in particular under the trade name Uvinul N539 by BASF;
   - Etocrylene sold in particular under the trade name Uvinul N35 by BASF,
Benzophenone derivatives:
   - Benzophenone-1, sold under the trade name Uvinul 400 by BASF,
   - Benzophenone-2, sold under the trade name Uvinul D50 by BASF,
   - Benzophenone-3 or Oxybenzone, sold under the trade name Uvinul M40 by BASF,
   - Benzophenone-4, sold under the trade name Uvinul MS40 by BASF,
   - Benzophenone-5,
   - Benzophenone-6, sold under the trade name Helisorb 11 by Norquay,
   - Benzophenone-8, sold under the trade name Spectra-Sorb UV-24 by American Cyanamid,
   - Benzophenone-9, sold under the trade name Uvinul DS-49 by BASF,
   - Benzophenone-12,
Benzylidenecamphor derivatives:
   - 3-Benzylidenecamphor manufactured under the name Mexoryl SD by Chimex,
   - 4-Methylbenzylidenecamphor sold under the name Eusolex 6300 by Merck,
   - Benzylidenecamphorsulfonic acid, manufactured under the name Mexoryl SL by Chimex,
   - Camphor benzalkonium methosulfate manufactured under the name Mexoryl SO by Chimex,
   - Terephthalylidenedicamphorsulfonic acid manufactured under the name Mexoryl SX by Chimex,
   - Polyacrylamidomethylbenzylidenecamphor manufactured under the name Mexoryl SW by Chimex.
Phenylbenzimidazole derivatives:
   - Phenylbenzimidazolesulfonic acid sold especially under the trade name Eusolex 232 by Merck,
   - Benzimidazilate sold under the trade name Neo Heliopan AP by Haarmann & Reimer,
Triazine derivatives:
   - Anisotriazine sold under the trade name Tinosorb S by Ciba Speciality Chemicals,
   - Ethylhexyl triazone sold especially under the trade name Uvinul T150 by BASF,
   - Diethylhexyl butamidotriazone sold under the trade name Uvasorb HEB by Sigma 3V,
Phenylbenzotriazole derivatives:
   - Drometrizole trisiloxane sold under the name Silatrizole by Rhodia Chimie,
Anthranilic derivatives:
   - Menthyl anthranilate sold under the trade name Neo Heliopan MA by Haarmann & Reimer,
Imidazoline derivatives:
   - Ethylhexyl dimethoxybenzylidene dioxoimidazoline propionate,
Benzalmalonate derivatives:
   - Polyorganosiloxane with benzalmalonate function sold under the trade name Parsol SLX by Hoffmann LaRoche, and mixtures thereof.

The soluble organic UV-screening agents that are more particularly preferred are chosen from the following compounds:
- Ethylhexyl Salicylate,
- Butyl Methoxydibenzoylmethane,
- Ethylhexyl Methoxycinnamate,
- Octocrylene,
- Phenylbenzimidazole Sulfonic Acid,
- Terephthalylidene Dicamphor Sulfonic,
- Benzophenone-3,
- Benzophenone-4,
- Benzophenone-5,
- 4-Methylbenzylidenecamphor,
- Benzimidazilate,
- Anisotriazine,
- Ethylhexyl triazone,
- Diethylhexyl Butamido Triazone,
- Drometrizole Trisiloxane,
and mixtures thereof.

The supplementary soluble UV-screening agent(s) are generally present in concentrations ranging from 0.1% to 40% by weight approximately, and preferably from 0.2% to 20% by weight approximately, relative to the total weight of the composition.

The inorganic UV-screening agents used in accordance with the present invention are metal oxide pigments. More preferentially, the inorganic UV-screening agents of the invention are metal oxide particles with an average elementary particle size of less than or equal to 0.5 µm, more preferentially between 0.005 and 0.5 µm, even more preferentially between 0.01 and 0.2 µm, better still between 0.01 and 0.1 µm and more particularly preferentially between 0.015 and 0.05 µm.

They may be chosen in particular from titanium oxide, zinc oxide, iron oxide, zirconium oxide and cerium oxide, or mixtures thereof.

Such coated or uncoated metal oxide pigments are described in particular in patent application EP-A-0 518 773. Commercial pigments that may be mentioned include the products sold by the companies Sachtleben Pigments, Tayca, Merck and Degussa.

The metal oxide pigments may be coated or uncoated.

The coated pigments are pigments that have undergone one or more surface treatments of chemical, electronic, mechanochemical and/or mechanical nature with compounds such as amino acids, beeswax, fatty acids, fatty alcohols, anionic surfactants, lecithins, sodium, potassium, zinc, iron or aluminium salts of fatty acids, metal alkoxides (of titanium or aluminium), polyethylene, silicones, proteins (collagen, elastin), alkanolamines, silicon oxides, metal oxides or sodium hexametaphosphate.

The coated pigments are more particularly titanium oxides that have been coated:
- with silica, such as the product Sunveil from the company Ikeda,
- with silica and iron oxide, such as the product Sunveil F from the company Ikeda,
- with silica and alumina, such as the products Microtitanium Dioxide MT 500 SA and Microtitanium Dioxide MT 100 SA from the company Tayca and Tioveil from the company Tioxide,
- with alumina, such as the products Tipaque TTO-55 (B) and Tipaque TTO-55 (A) from the company Ishihara, and UVT 14/4 from the company Sachtleben Pigments,
- with alumina and aluminium stearate, such as the products Microtitanium Dioxide MT 100 T, MT 100 TX, MT 100 Z and MT-01 from the company Tayca, the products Solaveil CT-10 W and Solaveil CT 100 from the company Uniqema and the product Eusolex T-AVO from the company Merck,
- with silica, alumina and alginic acid, such as the product MT-100 AQ from the company Tayca,
- with alumina and aluminium laurate, such as the product Microtitanium Dioxide MT 100 S from the company Tayca,
- with iron oxide and iron stearate, such as the product Microtitanium Dioxide MT 100 F from the company Tayca,
- with zinc oxide and zinc stearate, such as the product BR 351 from the company Tayca,
- with silica and alumina and treated with a silicone, such as the products Microtitanium Dioxide MT 600 SAS, Microtitanium Dioxide MT 500 SAS or Microtitanium Dioxide MT 100 SAS from the company Tayca,
- with silica, alumina and aluminum stearate and treated with a silicone, such as the product STT-30-DS from the company Titan Kogyo,
- with silica and treated with a silicone, such as the product UV-Titan X 195 from the company Sachtleben Pigments,
- with alumina and treated with a silicone, such as the products Tipaque TTO-55 (S) from the company Ishihara or UV Titan M 262 from the company Sachtleben Pigments,
- with triethanolamine, such as the product STT-65-S from the company Titan Kogyo,
- with stearic acid, such as the product Tipaque TTO-55 (C) from the company Ishihara,
- with sodium hexametaphosphate, such as the product Microtitanium Dioxide MT 150 W from the company Tayca,
- TiO₂ treated with octyltrimethylsilane, sold under the trade name T 805 by the company Degussa Silices,
- TiO₂ treated with a polydimethylsiloxane, sold under the trade name 70250 Cardre UF TiO2SI3 by the company Cardre,
- anatase/rutile TiO₂ treated with a polydimethylhydrogenosiloxane, sold under the trade name Microtitanium Dioxide USP Grade Hydrophobic by the company Color Techniques.

Mention may also be made of TiO₂ pigments doped with at least one transition metal such as iron, zinc or manganese and more particularly manganese. Preferably, said doped pigments are in the form of an oily dispersion. The oil present in the oily dispersion is preferably chosen from triglycerides including those of capric/caprylic acids. The oily dispersion of titanium oxide particles may also comprise one or more dispersants, for instance a sorbitan ester, for instance sorbitan isostearate, or a polyoxyalkylenated fatty acid ester of glycerol, for instance TRI-PPG-3 myristyl ether citrate and polyglyceryl-3 polyricinoleate. Preferably, the oily dispersion of titanium oxide particles comprises at least one dispersant chosen from polyoxyalkylenated fatty acid esters of glycerol. Mention may be made more particularly of the oily dispersion of TiO₂ particles doped with manganese in capric/caprylic acid triglyceride in the presence of TRI-PPG-3 myristyl ether citrate and polyglyceryl-3 polyricinoleate and sorbitan isostearate having the INCI name: titanium dioxide (and) TRI-PPG-3 myristyl ether citrate (and) polyglyceryl-3 ricinoleate (and) sorbitan isostearate, for instance the product sold under the trade name Optisol TD50 by the company Croda.

The uncoated titanium oxide pigments are sold, for example, by the company Tayca under the trade names Microtitanium Dioxide MT 500 B or Microtitanium Dioxide MT 600 B, by the company Degussa under the name P 25, by the company Wackher under the name Transparent Titanium Oxide PW, by the company Miyoshi Kasei under the name UFTR, by the company Tomen under the name ITS and by the company Tioxide under the name Tioveil AQ.

The uncoated zinc oxide pigments are, for example:
- those sold under the name Z-Cote by the company Sunsmart;
- those sold under the name Nanox by the company Elementis;
- those sold under the name Nanogard WCD 2025 by the company Nanophase Technologies.

The coated zinc oxide pigments are, for example:
- those sold under the name Zinc Oxide CS-5 by the company Toshibi (ZnO coated with polymethylhydrosiloxane);
- those sold under the name Nanogard Zinc Oxide FN by the company Nanophase Technologies (as a 40% dispersion in Finsolv TN, C12-C15 alkyl benzoate);
- those sold under the name Daitopersion Zn-30 and Daitopersion Zn-50 by the company Daito (dispersions in cyclopolymethylsiloxane/oxyethylenated polydimethylsiloxane, containing 30% or 50% of zinc oxides coated with silica and polymethylhydrogenosiloxane);
- those sold under the name NFD Ultrafine ZnO by the company Daikin (ZnO coated with perfluoroalkyl phosphate and copolymer based on perfluoroalkylethyl as a dispersion in cyclopentasiloxane);
- those sold under the name SPD-Z1 by the company Shin-Etsu (ZnO coated with silicone-grafted acrylic polymer, dispersed in cyclodimethylsiloxane);
- those sold under the name Escalol Z100 by the company ISP (alumina-treated ZnO dispersed in an ethylhexyl methoxycinnamate/PVP-hexadecene copolymer/methicone mixture);
- those sold under the name Fuji ZnO-SMS-10 by the company Fuji Pigment (ZnO coated with silica and polymethylsilsesquioxane);
- those sold under the name Nanox Gel TN by the company Elementis (ZnO dispersed at 55% in C12-C15 alkyl benzoate with hydroxystearic acid polycondensate).

The uncoated cerium oxide pigments may, for example, be those sold under the name Colloidal Cerium Oxide by the company Rhône-Poulenc.

The non-coated iron oxide pigments are sold, for example, by the company Arnaud under the names Nanogard WCD 2002 (FE 45B), Nanogard Iron FE 45 BL AQ, Nanogard FE 45R AQ and Nanogard WCD 2006 (FE 45R) or by the company Mitsubishi under the name TY-220.

The coated iron oxide pigments are sold, for example, by the company Arnaud under the names Nanogard WCD 2008 (FE 45B FN), Nanogard WCD 2009 (FE 45B 556), Nanogard FE 45 BL 345 and Nanogard FE 45 BL or by the company BASF under the name Transparent Iron Oxide.

Mention may also be made of mixtures of metal oxides, in particular of titanium dioxide and of cerium dioxide, including the equal-weight mixture of titanium dioxide and cerium dioxide coated with silica, sold by the company Ikeda under the name Sunveil A, and also the mixture of titanium dioxide and zinc dioxide coated with alumina, silica and silicone, such as the product M 261 sold by the company Sachtleben Pigments, or coated with alumina, silica and glycerol, such as the product M 211 sold by the company Sachtleben Pigments.

According to the invention, coated or uncoated titanium oxide pigments are particularly preferred.

The inorganic UV-screening agents are preferably present in the compositions according to the invention in a content ranging from 0.1% to 30% by weight, more particularly from 0.5% to 20% by weight and in particular from 1% to 10% by weight, relative to the total weight of the composition.

According to the invention, the compositions according to the invention comprise an aqueous phase and/or an oily phase.

### OILY PHASE

The compositions in accordance with the invention comprise at least one oily phase.

For the purposes of the invention, the term "oily phase" is intended to mean a phase comprising at least one oil and all of the liposoluble and lipophilic ingredients and the fatty substances used for the formulation of the compositions of the invention.

The term "oil" is intended to mean any fatty substance that is in liquid form at ambient temperature (20-25°C) and atmospheric pressure (760 mmHg).

An oil that is suitable for use in the invention may be volatile or non-volatile.

An oil that is suitable for use in the invention may be chosen from hydrocarbon-based oils, silicone oils and fluoro oils, and mixtures thereof.

A hydrocarbon-based oil that is suitable for use in the invention may be an animal hydrocarbon-based oil, a plant hydrocarbon-based oil, a mineral hydrocarbon-based oil or a synthetic hydrocarbon-based oil.

An oil that is suitable for use in the invention may be advantageously chosen from mineral hydrocarbon-based oils, plant hydrocarbon-based oils, synthetic hydrocarbon-based oils and silicone oils, and mixtures thereof.

For the purposes of the present invention, the term "silicone oil" is intended to mean an oil comprising at least one silicon atom, and especially at least one Si-O group.

The term "hydrocarbon-based oil" is intended to mean an oil mainly containing hydrogen and carbon atoms.

The term "fluoro oil" refers to an oil comprising at least one fluorine atom.

A hydrocarbon-based oil that is suitable for use in the invention may also optionally comprise oxygen, nitrogen, sulfur and/or phosphorus atoms, for example in the form of hydroxyl, amine, amide, ester, ether or acid groups, and in particular in the form of hydroxyl, ester, ether or acid groups.

The oily phase generally comprises, in addition to the lipophilic UV-screening agent(s), at least one volatile or non-volatile hydrocarbon-based oil and/or one volatile and/or non-volatile silicone oil.

For the purposes of the invention, the term "volatile oil" is intended to mean an oil that is capable of evaporating on contact with the skin or the keratin fibre in less than one hour, at ambient temperature and atmospheric pressure. The volatile oil(s) of the invention are volatile cosmetic oils which are liquid at ambient temperature and which have a non-zero vapour pressure, at ambient temperature and atmospheric pressure, ranging in particular from 0.13 Pa to 40 000 Pa (10⁻³ to 300 mmHg), in particular ranging from 1.3 Pa to 13 000 Pa (0.01 to 100 mmHg) and more particularly ranging from 1.3 Pa to 1300 Pa (0.01 to 10 mmHg).

The term "non-volatile oil" is intended to mean an oil that remains on the skin or the keratin fibre at ambient temperature and atmospheric pressure for at least several hours, and that in particular has a vapour pressure of less than 10⁻³ mmHg (0.13 Pa).

### Hydrocarbon-based oils

Mention may especially be made, as non-volatile hydrocarbon-based oils which may be used according to the invention, of:
(i) hydrocarbon-based oils of plant origin, such as glyceride triesters, which are generally triesters of fatty acids and of glycerol, the fatty acids of which can have varied chain lengths from C₄ to C₂₄, it being possible for these chains to be saturated or unsaturated and linear or branched; these oils are in particular wheatgerm oil, sunflower oil, grape seed oil, sesame oil, corn oil, apricot oil, castor oil, shea oil, avocado oil, olive oil, soybean oil, sweet almond oil, palm oil, rapeseed oil, cottonseed oil, hazelnut oil, macadamia oil, jojoba oil, alfalfa oil, poppy oil, pumpkin oil, sesame oil, marrow oil, blackcurrant oil, evening primrose oil, millet oil, barley oil, quinoa oil, rye oil, safflower oil, candlenut oil, passionflower oil and musk rose oil; or also caprylic/capric acid triglycerides, such as those sold by the company Stéarineries Dubois or those sold under the names Miglyol 8100, 8120 and 818^{®} by the company Dynamit Nobel;
(ii) synthetic ethers having from 10 to 40 carbon atoms;
(iii) linear or branched hydrocarbons of mineral or synthetic origin, such as petroleum jelly, polydecenes, hydrogenated polyisobutene, such as Parleam, squalane and mixtures thereof;
(iv) synthetic esters, for instance oils of formula RCOOR' in which R represents the linear or branched fatty acid residue containing from 1 to 40 carbon atoms and R' represents a hydrocarbon-based chain that is in particular branched, containing from 1 to 40 carbon atoms, on condition that R + R' is ≥ 10, for instance purcellin oil (cetostearyl octanoate), isopropyl myristate, isopropyl palmitate, C₁₂-C₁₅ alkyl benzoate, such as the product sold under the trade name Finsolv TN^{®} or Witconol TN^{®} by the company Witco or Tegosoft TN^{®} by the company Evonik Goldschmidt, 2-ethylphenyl benzoate, such as the commercial product sold under the name X-Tend 226^{®} by the company ISP, isopropyl lanolate, hexyl laurate, diisopropyl adipate, isononyl isononanoate, oleyl erucate, 2-ethylhexyl palmitate, isostearyl isostearate, diisopropyl sebacate, such as the product sold under the name Dub Dis by the company Stearinerie Dubois, octanoates, decanoates or ricinoleates of alcohols or polyalcohols, such as propylene glycol dioctanoate; hydroxylated esters, such as isostearyl lactate or diisostearyl malate; and pentaerythritol esters; citrates or tartrates, such as di(linear C₁₂-C₁₃ alkyl) tartrates, such as those sold under the name Cosmacol ETI^{®} by the company Enichem Augusta Industriale, and also di(linear C₁₄-C₁₅ alkyl) tartrates, such as those sold under the name Cosmacol ETL^{®} by the same company; or acetates;
(v) fatty alcohols which are liquid at ambient temperature and which have a branched and/or unsaturated carbon chain having from 12 to 26 carbon atoms, such as octyldodecanol, isostearyl alcohol, oleyl alcohol, 2-hexyldecanol, 2-butyloctanol or 2-undecylpentadecanol;
(vi) higher fatty acids, such as oleic acid, linoleic acid or linolenic acid;
(vii) carbonates, such as dicaprylyl carbonate, such as the product sold under the name Cetiol CC^{®} by the company Cognis;
(viii) fatty amides, such as isopropyl N-lauroyl sarcosinate, such as the product sold under the trade name Eldew SL205^{®} from the company Ajinomoto, and mixtures thereof.

Among the non-volatile hydrocarbon-based oils that may be used according to the invention, preference will be given more particularly to glyceride triesters and in particular to caprylic/capric acid triglycerides, synthetic esters and in particular isononyl isononanoate, oleyl erucate, C₁₂-C₁₅ alkyl benzoate, 2-ethylphenyl benzoate and fatty alcohols, in particular octyldodecanol.

As volatile hydrocarbon-based oils that may be used according to the invention, mention may be made in particular of hydrocarbon-based oils containing from 8 to 16 carbon atoms and in particular of branched C₈-C₁₆ alkanes, such as C₈-C₁₆ isoalkanes of petroleum origin (also known as isoparaffins), such as isododecane (also known as 2,2,4,4,6-pentamethylheptane), isodecane or isohexadecane, the oils sold under the Isopar or Permethyl trade names, branched C₈-C₁₆ esters, isohexyl neopentanoate, and mixtures thereof.

Mention may also be made of the alkanes described in the Cognis patent applications WO 2007/068 371 or WO 2008/155 059 (mixtures of distinct alkanes differing by at least one carbon). These alkanes are obtained from fatty alcohols, which are themselves obtained from coconut or palm oil. Mention may be made of the mixtures of n-undecane (C₁₁) and n-tridecane (C₁₃) obtained in Examples 1 and 2 of patent application WO 2008/155 059 from the company Cognis. Mention may also be made of n-dodecane (C₁₂) and n-tetradecane (C₁₄) sold by Sasol under the respective references Parafol 12-97 and Parafol 14-97^{®}, and also mixtures thereof.

Other volatile hydrocarbon-based oils, for instance petroleum distillates, in particular those sold under the name Shell Solt^{®} by the company Shell, may also be used. According to one embodiment, the volatile solvent is chosen from volatile hydrocarbon oils having from 8 to 16 carbon atoms and mixtures thereof.

### b) Silicone oils

The non-volatile silicone oils can be chosen in particular from non-volatile polydimethylsiloxanes (PDMSs), polydimethylsiloxanes comprising alkyl or alkoxy groups which are pendent and/or at the end of the silicone chain, which groups each have from 2 to 24 carbon atoms, or phenylated silicones, such as phenyl trimethicones, phenyl dimethicones, phenyl(trimethylsiloxy)diphenylsiloxanes, diphenyl dimethicones, diphenyl(methyldiphenyl)trisiloxanes or (2-phenylethyl)trimethylsiloxysilicates.

Examples of volatile silicone oils that may be mentioned include volatile linear or cyclic silicone oils, in particular those with a viscosity ≤ 8 centistokes (8 × 10⁻⁶ m²/s) and in particular having from 2 to 7 silicon atoms, these silicones optionally comprising alkyl or alkoxy groups having from 1 to 10 carbon atoms. As volatile silicone oils that may be used in the invention, mention may be made especially of octamethylcyclotetrasiloxane, decamethylcyclopentasiloxane, dodecamethylcyclohexasiloxane, heptamethylhexyltrisiloxane, heptamethyloctyltrisiloxane, hexamethyldisiloxane, octamethyltrisiloxane, decamethyltetrasiloxane and dodecamethylpentasiloxane, and mixtures thereof.

Mention may also be made of the volatile linear alkyltrisiloxane oils of general formula (I): 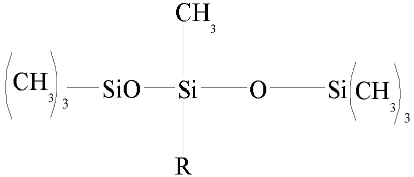 where R represents an alkyl group comprising from 2 to 4 carbon atoms, one or more hydrogen atoms of which may be replaced with a fluorine or chlorine atom.

Among the oils of general formula (I), mention may be made of:
3-butyl-1,1,1,3,5,5,5-heptamethyltrisiloxane,
3-propyl-1,1,1,3,5,5,5-heptamethyltrisiloxane, and
3-ethyl-1,1,1,3,5,5,5-heptamethyltrisiloxane,
corresponding to the oils of formula (I) for which R is, respectively, a butyl group, a propyl group or an ethyl group.

### Fluoro oils

Use may also be made of volatile fluoro oils, such as nonafluoromethoxybutane, decafluoropentane, tetradecafluorohexane, dodecafluoropentane, and mixtures thereof.

An oily phase according to the invention may also comprise other fatty substances, mixed with or dissolved in the oil.

Another fatty substance that may be present in the oily phase may be, for example:
- a fatty acid chosen from fatty acids comprising from 8 to 30 carbon atoms, such as stearic acid, lauric acid, palmitic acid and oleic acid;
- a wax chosen from waxes such as lanolin, beeswax, carnauba or candelilla wax, paraffin waxes, lignite waxes, microcrystalline waxes, ceresin or ozokerite, or synthetic waxes, such as polyethylene waxes or Fischer-Tropsch waxes;
- a gum chosen from silicone gums (dimethiconol);
- a pasty compound, such as polymeric or non-polymeric silicone compounds, esters of a glycerol oligomer, arachidyl propionate, fatty acid triglycerides and derivatives thereof;
- and mixtures thereof.

Preferentially, the overall oily phase, including all the lipophilic substances of the composition capable of being dissolved in this same phase, represents from 5% to 95% by weight and preferentially from 10% to 80% by weight, relative to the total weight of the composition.

### AQUEOUS PHASE

The compositions according to the invention comprise at least one aqueous phase.

The aqueous phase contains water and optionally other water-soluble or water-miscible organic solvents.

An aqueous phase that is suitable for use in the invention may comprise, for example, a water chosen from a natural spring water, such as water from La Roche-Posay, water from Vittel or waters from Vichy, or a floral water.

The water-soluble or water-miscible solvents that are suitable for use in the invention comprise short-chain monoalcohols, for example C₁-C₄ monoalcohols, such as ethanol or isopropanol; diols or polyols, such as ethylene glycol, 1,2-propylene glycol, 1,3-butylene glycol, pentylene glycol, hexylene glycol, diethylene glycol, dipropylene glycol, 2-ethoxyethanol, diethylene glycol monomethyl ether, triethylene glycol monomethyl ether, glycerol and sorbitol, and mixtures thereof.

According to a preferred embodiment, use may more particularly be made of ethanol, propylene glycol, glycerol, and mixtures thereof.

According to one particular form of the invention, the overall aqueous phase, including all the hydrophilic substances of the composition which are capable of being dissolved in this same phase, represents from 5% to 95% by weight and preferably from 10% to 80% by weight relative to the total weight of the composition.

The compositions according to the invention can also comprise agents for the artificial tanning and/or browning of the skin (self-tanning agents), such as for example dihydroxyacetone (DHA).

The compositions of the invention can also comprise conventional cosmetic adjuvants, in particular chosen from fatty substances, organic solvents, ionic or non-ionic thickeners, demulcents, antioxidants, free-radical scavengers, opacifiers, stabilizers, emollients, silicones, α-hydroxy acids, anti-foams, moisturizing agents, vitamins, insect repellents, fragrances, preservatives, surfactants, fillers, photoprotective agents, polymers other than those of the invention, propellants, basifying or acidifying agents, dyes or any other ingredient normally used in cosmetics, in particular for the production of anti-sun compositions, or of care or makeup compositions, in particular in the form of emulsions.

The fatty substances can be constituted of an oil or a wax or mixtures thereof, and they also comprise fatty acids, fatty alcohols and fatty acid esters. The oils can be chosen from animal, plant, mineral or synthetic oils and in particular from liquid petroleum jelly, paraffin oil, volatile or non-volatile silicone oils, isoparaffins, polyolefins, and fluoro and perfluoro oils. Likewise, the waxes may be chosen from animal, fossil, plant, mineral or synthetic waxes known per se.

Mention may be made, among organic solvents, of lower alcohols and polyols.

Needless to say, those skilled in the art will take care to select this or these optional additional compound(s) and/or the amounts thereof so that the advantageous properties, in particular the persistence with respect to water and the stability, intrinsically associated with the compositions in accordance with the invention are not, or not substantially, adversely affected by the envisaged addition(s).

The compositions of the invention can be prepared according to the techniques well known to those skilled in the art, in particular those intended for the preparation of oil-in water or water-in-oil emulsions.

These compositions may be in particular in the form of a simple or complex emulsion (O/W, W/O, O/W/O or W/O/W) such as a cream, a milk or a cream-gel, a powder or a solid rod, and optionally be packaged as an aerosol, and be in the form of a foam or a spray.

When it is an emulsion, the aqueous phase of this emulsion may comprise a non-ionic vesicular dispersion prepared according to known processes (Bangham, Standish and Watkins, J. Mol. Biol. 13, 238 (1965), FR 2 315 991 and FR 2 416 008).

The cosmetic composition of the invention can be used as a protective composition for the human epidermis or the hair against ultraviolet rays, as daily photoprotection or as an anti-sun composition, or a makeup product.

When the cosmetic composition according to the invention is used for the protection of the human epidermis against UV rays, or as an anti-sun composition, it can be in the form of a suspension or a dispersion in solvents or fatty substances, in the form of a non-ionic vesicular dispersion or else in the form of an emulsion, such as a cream or milk, or in the form of an ointment, gel, a gel-cream, a solid rod, a powder, a stick, an aerosol foam or a spray.

When the cosmetic composition according to the invention is used for protecting the hair against UV rays, it can be in the form of a shampoo, a lotion, a gel, an emulsion, or a non-ionic vesicular dispersion and can constitute, for example, a rinse-off composition to be applied before or after shampooing, before or after dyeing or bleaching, before, during or after permanent-waving or relaxing of the hair, a styling or treating lotion or gel, a blow drying or hair setting lotion or gel, or a composition for permanent-waving or relaxing, dyeing or bleaching of the hair.

When the composition is used as a product for making up the eyelashes, the eyebrows or the skin, such as a cream for treating the epidermis, a foundation, a lipstick, an eyeshadow, a face powder, a mascara or an eyeliner, it can be in solid or pasty form, such as oil-in-water or water-in-oil emulsions, non-ionic vesicular dispersions or else suspensions.

By way of indication, for the anti-sun formulations in accordance with the invention which have a support of oil-in-water emulsion type, the aqueous phase (comprising in particular the hydrophilic screening agents) generally represents from 40% to 95% by weight, preferably from 70% to 90% by weight, relative to the whole of the formulation, the oily phase (comprising in particular the lipophilic screening agents) represents from 5% to 60% by weight, preferably from 10% to 40% by weight, relative to the whole of the formulation, and the (co)emulsifier(s) represent from 0.5% to 10% by weight, preferably from 2% to 5% by weight, relative to the whole of the formulation.

The compositions according to the invention find their application in a large number of treatments, in particular cosmetic treatments, for the skin, the lips and the hair, including the scalp, in particular for protecting and/or caring for the skin, the lips and/or the hair, and/or for making up the skin and/or the lips.

Another subject of the present invention is constituted of the use of the compositions according to the invention as defined above for the manufacture of products for the cosmetic treatment of the skin, the lips, the nails, the hair, the eyelashes, the eyebrows and/or the scalp, in particular care products, antisun products and makeup products.

The cosmetic compositions according to the invention may be used, for example, as makeup products.

The cosmetic compositions according to the invention may be used, for example, as care products and/or antisun products for the face and/or body, with a liquid to semi-liquid consistency, such as milks, more or less smooth creams, cream gels or pastes. They may optionally be packaged in aerosol form and may be in the form of a mousse or a spray.

The compositions according to the invention in the form of vaporizable fluid lotions in accordance with the invention are applied to the skin or hair in the form of fine particles by means of pressurizing devices. The devices in accordance with the invention are well known to those skilled in the art and comprise non-aerosol pumps or "atomizers", aerosol containers comprising a propellant and aerosol pumps using compressed air as propellant. These devices are described in patents US 4 077 441 and US 4 850 517.

The compositions packaged in aerosol form in accordance with the invention generally contain conventional propellants, for instance hydrofluoro compounds, dichlorodifluoromethane, difluoroethane, dimethyl ether, isobutane, n-butane, propane or trichlorofluoromethane. They are preferably present in amounts ranging from 15% to 50% by weight relative to the total weight of the composition.

### ASSEMBLY

According to another aspect, the invention also relates to a cosmetic assembly comprising:
i) a container delimiting one or more compartment(s), said container being closed by a closing member and optionally being unsealed; and
ii) a makeup and/or care composition in accordance with the invention placed inside said compartment(s).

The container can, for example, be in the form of a pot or a case.

The closing member can be in the form of a lid comprising a cap mounted so as to be able to move by translation or by pivoting relative to the container housing said makeup and/or care composition(s).

The examples that follow serve to illustrate the invention. In these examples, the amounts of the composition ingredients are given as weight percentages relative to the total weight of the composition.

### EXAMPLES:

### Exemple of preparation of polymer 1:

### Determination of the molecular weight by gel permeation chromatography (GPC):

The sample is prepared by preparing a solution of the polymer at 10 mg/ml in tetrahydrofuran. The sample is placed in an oven at 54°C for 10 minutes and then in an oscillating shaker for 60 minutes to aid dissolution. After visual inspection, the sample appears to be totally dissolved in the solvent.

The sample prepared was analysed using two polypore 300×7.5 mm columns (manufactured by Agilent Technologies), a Waters 2695 chromatographic system, a tetrahydrofuran mobile phase and detection by refractive index. The sample was filtered through a 0.45 µm nylon filter, before being injected into the liquid chromatograph. The standards used for the calibration are the Easi Vial narrow polystyrene (PS) standards from Agilent Technologies.

Polystyrene standards ranging from 2 520 000 to 162 daltons were used for the calibration.

The system is equipped with a PSS SECcurity 1260 RI detector. The polystyrene calibration curve was used to determine the average molecular weight. The recording of the diagrams and the determination of the various molecular weights were performed by the Win GPC Unichrom 81 program.

### Determination of the melting point by differential scanning calorimetry (or DSC):

This method describes the general procedure for determining the melting point of polymers by differential scanning calorimetry. This method is based on the standards ASTM E791 and ASTM D 34182 and the DSC calibration is performed according to standard ASTM E 9672.

### Behenyl acrylate / 2-hydroxyethyl acrylate copolymer (Polymer 1):

In a 4-necked flask equipped with side-blade mixer, an internal thermometer, two funnels, a reflux condenser, and an extension for two other necks, 175 g of behenyl acrylate, 25 g of 2-hydroxyethyl acrylate and 0.4 g of 2,2'-azobis(2-methylbutyronitrile) (Akzo Nobel) were added, over the course of 60 minutes at 80°C, to 40 g of isopropanol, with stirring, after having removed the oxygen from the system by means of a nitrogen flush for 20 minutes. The mixture was stirred at 80°C for 3 hours. The solvent was then eliminated by vacuum distillation, then 1 g of dilauryl peroxide was added and the reaction was continued for 60 minutes at 110°C. The step was repeated. The mixture was then cooled to 90°C, a stream of demineralized water was added and the mixture was then stirred. The water was removed by vacuum distillation.
Molecular weight: Mn = 7300 g/mol , Mw = 21 000 , Mw/Mn = 2.8
Melting point: 65°C

### Formulation examples

### EXAMPLES 1 to 8

For each composition, the stability over time was studied at various temperatures and/or the sensory aspect was evaluated during and after its application to the skin.

### Study of the stability over time at various temperatures

The stability is studied over time by observing the change in the composition with regard to its macroscopic appearance and its microscopic appearance, at various temperatures such as ambient temperature (AT), 4°C or 45°C.

The following compositions were prepared.

| | INCI name | Ex 1 | Ex 2 | Ex 3 | Ex 4 | Ex 5* | Ex 6* | Ex 7* | Ex 8* |
|---|---|---|---|---|---|---|---|---|---|
| A1 | Behenyl Alcohol (and) Glyceryl Stearate (and) Disodium Ethylene Dicocamide PEG-15 Disulfate (and) Glyceryl Stearate Citrate | 3 | 3 | 3 | 3 | 3 | 3 | 3 | 3 |
| A1 | Cetyl alcohol | 0.5 | 0.5 | 0.5 | 0.5 | 0.5 | 0.5 | 0.5 | 0.5 |
| A1 | Octocrylene | 5 | 5 | 5 | 5 | 5 | 5 | 5 | 5 |
| A1 | Butyl Methoxydibenzoylmethane | 5 | 5 | 5 | 5 | 5 | 5 | 5 | 5 |
| A1 | Ethylhexyl salicylate | 5 | 5 | 5 | 5 | 5 | 5 | 5 | 5 |
| A1 | Caprylyl glycol | 0.4 | 0.4 | 0.4 | 0.4 | 0.4 | 0.4 | 0.4 | 0.4 |
| A2 | Phenoxyethanol | 0.6 | 0.6 | 0.6 | 0.6 | 0.6 | 0.6 | 0.6 | 0.6 |
| B | Disodium EDTA | 0.1 | 0.1 | 0.1 | 0.1 | 0.1 | 0.1 | 0.1 | 0.1 |
| C | Ammonium Polyacryloyldimethyl Taurate (Hostacerin AMPS^{®}) | 0.7 | 0.7 | 0.7 | 0.7 | 0.7 | 0.7 | 0.7 | 0.7 |
| C | Xanthan gum | 0.25 | 0.25 | 0.25 | 0.25 | 0.25 | 0.25 | 0.25 | 0.25 |
| A1 | Behenyl acrylate / 2-hydroxyethyl acrylate copolymer (Polymer 1) as previously synthesized | | | | | 2 | 2 | 2 | 2 |
| A1 | Polystearyl acrylate (IPA-13-1) | 2 | 2 | 2 | 2 | | | | |
| D | Methylene bisbenzotrazolyl tetramethylbutylphenol (and) Polyglyceryl-10 laurate (concentration of active material) | 6 | 3 | 1 | | 6 | 3 | 1 | |
| D | Methylene bisbenzotriazolyl tetramethylbutylphenol (and) Decyl glucoside (Tinosorb M) (concentration of active material) | | | | 6 | | | | 6 |
| B | Water (qs) | 100% | 100 | 100 | 100 | 100 | 100 | 100 | 100 |

### PROCEDURE:

Dissolve the starting materials of the fatty phase (A1) at 75/80°C.
Dissolve the starting materials of the aqueous phase (B) at AT.
Add the phenoxyethanol (A2) to A1 just before the emulsification.
At 65°C, emulsify B in (A1 + A2), turbine (3000 rpm) + paddles for 10 min.
Add the gelling agents (C), turbine (3000 rpm) + paddles for 10 min.
Add the remaining water (D), turbine (3000 rpm) + paddles for 10 min while beginning to cool to AT.
Continue the cooling with paddles.
At AT, add the insoluble screening agent (E), then operate the turbine at 3000 rpm, then stir with paddles for 5 min.

### Results

The properties of glide on application and of softness after application were compared for each of the compositions at equivalent screening-agent concentration.
50 µl of product sampled with a pipette are deposited on the back of the hand (or on SkinFX (reconstructed skin)).
15 circular motions are performed on the back of the hand over the course of 15 seconds.

After having waited 15 seconds, 15 circular motions are again performed over the course of 15 seconds.

Trained experts evaluate from 1 to 5 with an increment of 0.5 (1 = negative limit, 5 = positive limit).
**Glide**: Tactile evaluation at the end of application. Slide 2 fingers on the skin.
**Tack:** Tactile evaluation. Tactile evaluation 45 s after application.

| composition | Ex 1 | Ex 5* |
|---|---|---|
| Glide on application | 1 | 2 |
| Softness after application | 2 | 3 |

| composition | Ex 2 | Ex 6* |
|---|---|---|
| Glide on application | 1 | 2 |
| Softness after application | 3 | 4 |

| composition | Ex 3 | Ex 7* |
|---|---|---|
| Glide on application | 2 | 3 |
| Softness after application | 4 | 5 |

| composition | Ex 4 | Ex 8* |
|---|---|---|
| Glide on application | 1 | 2 |
| Softness after application | 2 | 3 |

The compositions comprising an insoluble screening agent and a polymer b) according to the invention have improved cosmetic properties.

### EXAMPLES 9 to 14

The following compositions were prepared.

| INCI name (EU) | Ex 9 | Ex 10 | Ex 11 | Ex 12* | Ex 13* | Ex14* |
|---|---|---|---|---|---|---|
| Homosalate | 7 | 7 | 7 | 7 | 7 | 7 |
| Octocrylene | 5 | 5 | 5 | 5 | 5 | 5 |
| Butyl Methoxydibenzoylmethane | 5 | 5 | 5 | 5 | 5 | 5 |
| Ethylhexyl salicylate | 5 | 5 | 5 | 5 | 5 | 5 |
| Ethylhexyl triazone | 1.5 | 1.5 | 1.5 | 1.5 | 1.5 | 1.5 |
| C12-15 Alkyl benzoate | 4 | 4 | 4 | 4 | 4 | 4 |
| Caprylyl glycol | 0.4 | 0.4 | 0.4 | 0.4 | 0.4 | 0.4 |
| Phenoxyethanol | 0.6 | 0.6 | 0.6 | 0.6 | 0.6 | 0.6 |
| Disodium EDTA | 0.1 | 0.1 | 0.1 | 0.1 | 0.1 | 0.1 |
| Acrylates copolymer | 1.2 | 1.2 | 1.2 | 1.2 | 1.2 | 1.2 |
| Triethanolamine | 0.15 | 0.15 | 0.15 | 0.15 | 0.15 | 0.15 |
| Ammonium acryloyldimethyltaurate/VP copolymer | 0.4 | 0.4 | 0.4 | 0.4 | 0.4 | 0.4 |
| Behenyl acrylate / 2-hydroxyethyl acrylate | | | | 3 | 3 | 3 |
| copolymer (Polymer 1) as previously synthesized | | | | | | |
| Polystearyl acrylate (IPA-13-1) | 3 | 3 | 3 | | | |
| Methylene bisbenzotrazolyl tetramethylbutylphenol (and) Polyglyceryl-10 laurate (concentration of active material) | 6 | 3 | 1 | 6 | 3 | 1 |
| AQUA (qsp) | 100 | 100 | 100 | 100 | 100 | 100 |

| | | | | | | |
|---|---|---|---|---|---|---|
| *Example according to the invention | | | | | | |

### PROCEDURE:

Dissolve the SMs of the fatty phase (A1) at 75/80°C.
Dissolve the starting materials of the aqueous phase (B) at 65°C.
Add the phenoxyethanol (A2) to A1 just before the emulsification.
At 65°C, emulsify (A1 + A2) in B, turbine (3000 rpm) + paddles for 10 min.
Add the gelling agent (C), turbine (3000 rpm) + paddles for 10 min.
Initiate the cooling to AT while leaving the turbine (2000 rpm) and the paddles until T<40°C.
At AT, add the insoluble screening agent (D), turbine (3000 rpm) + paddles for 5 min.

### RESULTS

| | Ex 9 | Ex 10 | Ex 11 | **Ex 12*** | **Ex 13*** | **Ex 14*** |
|---|---|---|---|---|---|---|
| Macroscopic appearance 24 H | Granular | Granular | Granular | Smooth cream | Smooth cream | Smooth cream |
| Microscopic appearance 24 H | Broken emulsion | Broken emulsion | Broken emulsion | Fine and homogeneous emulsion | Fine and homogeneous emulsion | Fine and homogeneous emulsion |
| Change at 2 months AT and 45° | Broken emulsion | Broken emulsion | Broken emulsion | Fine and homogeneous emulsion | Fine and homogeneous emulsion | Fine and homogeneous emulsion |

The compositions according to the invention are stable, contrary to the compositions not containing the polymer b) according to the invention.

### EXAMPLES 15 to 17

The following compositions are prepared.

| INCI name (EU) | Ex15 | Ex16 | Ex17 |
|---|---|---|---|
| C12-15 Alkyl benzoate | 25 | 25 | 10 |
| Phenylene bis-benzoxazolyle in isobutene dispersion | - | - | 3%AM |
| Caprylyl glycol | 0.4 | 0.4 | 0.4 |
| Phenoxyethanol | 0.6 | 0.6 | 0.6 |
| Acrylates copolymer | 1.2 | 1.2 | 1.2 |
| Triethanolamine | 0.15 | 0.15 | 0.15 |
| Ammonium acryloyldimethyltaurateA/P copolymer | 0.4 | 0.4 | 0.4 |
| Behenyl acrylate / 2-hydroxyethyl acrylate copolymer (Polymer 1) as previously synthesized | 3 | 3 | 3 |
| Tris-biphenyl triazine in aqueous dispersion (Tinosorb A2B) | 3%AM | - | - |
| 1,1'-(1,4-piperazinediyl)bis[1-[2-[4-(diethylamino)-2-hydroxybenzoyl]phenyl]methanone in aqueous dispersion | - | 3%AM | - |
| Eau (qsp) | 100 % | 100 % | 100 % |

## Claims

1. Composition, in particular cosmetic composition, comprising at least one photoprotective system capable of screening out UV rays comprising at least one insoluble organic UV-screening agent a), **characterized in that** it also comprises at least one polymer b) comprising monomer units of formulae (A) and (B): 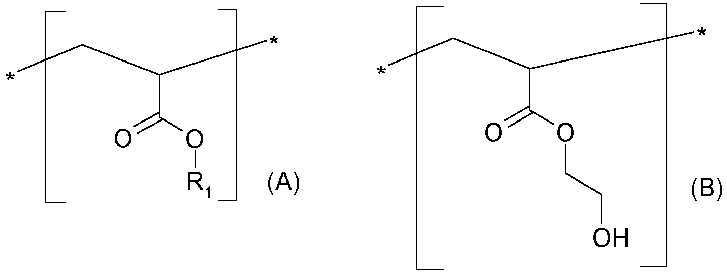 in which:
- R₁, independently of one another, is chosen from alkyl or alkylene radicals,
- and
- at least 60% by weight of the R₁ groups are behenyl radicals, the percentage by weight relating to the sum of all the R₁ groups present in the polymer,
- and
- the weight ratio of the sum of all the hydroxyethyl acrylate units to the sum of all the acrylate units bearing the R₁ group ranges from 1:30 to 1: 1,
- and the sum of the total of units A and B is at least 95% by weight of the total weight of the polymer,
- the polymer having a number-average molecular weight Mn ranging from 2000 to 9000 g/mol.

2. Composition according to Claim1, **characterized in that**, in the polymer b), R₁ is constituted of alkyl radicals, preferably of C₁₆-C₂₂ alkyl radicals, and more preferentially of behenyl (C₂₂) radicals.

3. Composition according to either one of Claims 1 to 2, **characterized in that**, in the polymer b), at least 70% by weight of the R₁ groups are behenyl radicals, preferentially at least 80% by weight, and more preferentially at least 90% by weight.

4. Composition according to any one of Claims 1 to 3, **characterized in that**, in the polymer b), all the R₁ groups are behenyl radicals.

5. Composition according to any one of Claims 1 to 4, **characterized in that**, in the polymer b), the weight ratio of the sum of all the hydroxyethyl acrylate units to the sum of all the acrylate units bearing the R₁ group ranges from 1:15 to 1:1, preferentially ranges from 1:10 to 1:4.

6. Composition according to any one of Claims 1 to 5, in which the polymer units present in the polymer b) are constituted of the units (A) and (B) described in any one of Claims 1 to 5.

7. Composition according to any one of Claims 1 to 6, **characterized in that** the polymer b) has a number-average molecular weight Mn ranging from 5000 to 9000 g/mol.

8. Composition according to any one of Claims 1 to 7, **characterized in that** the polymer b) has a melting point ranging from 60°C to 69°C, and preferentially ranging from 63°C to 67°C

9. Composition according to any one of Claims 1 to 8, **characterized in that** the polymer(s) b) can be present in the composition in an amount of active material ranging from 0.1% to 10% by weight, preferably from 0.2% to 4% by weight, relative to the total weight of the composition.

10. Composition according to any one of Claims 1 to 9, **characterized in that** the insoluble organic UV-screening agent is in the form of particles which have a mean size ranging from 0.01 to 5 µm and more preferentially from 0.01 to 2 µm and more particularly from 0.020 to 2 µm.

11. Composition according to any one of Claims 1 to 10, **characterized in that** the insoluble organic UV-screening agent is chosen in particular from organic UV-screening agents of the oxalanilide type, of the triazine type, of the benzotriazole type; of the vinylamide type; of the cinnamide type; of the type comprising one or more groups which are benzazole and/or benzofuran, benzothiophene or of the indole type; of the aryl vinylene ketone type; of the phenylene bis-benzoxazinone derivative type; of the amide, sulfonamide or acrylonitrile carbamate derivative type, or mixtures thereof.

12. Composition according to any one Claims 1 to 11, **characterized in that** the insoluble organic UV-screening agent is chosen from:
(i) symmetrical triazines substituted with naphthalenyl groups or polyphenyl groups, in particular 2,4,6-tris(diphenyl)triazine and/or 2,4,6-tris(ter-phenyl)triazine;
(ii) the methylenebis(hydroxyphenylbenzotriazole) compounds of formula (IV) below: 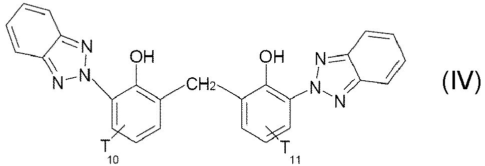 in which the radicals T₁₀ and T₁₁, which may be identical or different, denote a C₁-C₁₈ alkyl radical which may be substituted with one or more radicals chosen from C₁-C₄ alkyl, C₅-C₁₂ cycloalkyl or an aryl residue;
(iii) and mixtures thereof.

13. Composition according to Claim 12, **characterized in that** the methylenebis(hydroxyphenylbenzotriazole) compound of formula (IV) is in the form of an aqueous dispersion of particles having a mean particle size which ranges from 0.01 to 5 µm and more preferentially from 0.01 to 2 µm and more particularly from 0.020 to 2 µm, in the presence of at least one surfactant of structure CₙH₂ₙ₊₁ O(C₆H₁₀O₅)ₓH in which n is an integer from 8 to 16 and x is the mean degree of polymerization of the unit (C₆H₁₀O₅) and ranges from 1.4 to 1.6.

14. Composition according to Claim 11 or 12, **characterized in that** the methylenebis(hydroxyphenylbenzotriazole) compound of formula (IV) is in the form of an aqueous dispersion of particles having a mean particle size which ranges from 0.02 to 2 µm and more preferentially from 0.01 to 1.5 µm and more particularly from 0.02 to 1 µm in the presence of at least one mono(C₈-C₂₀)alkyl ester of polyglycerol having a degree of glycerol polymerization of at least 5.

15. Composition according to Claim 14, **characterized in that** the mono-(C₈-C₂₀)alkyl ester of polyglycerol is chosen from decaglyceryl caprate, decaglyceryl laurate, decaglyceryl myristate, decaglyceryl oleate, decaglyceryl stearate, decaglyceryl isostearate, hexaglyceryl caprate, hexaglyceryl laurate, hexaglyceryl myristate, hexaglyceryl oleate, hexaglyceryl stearate, hexaglyceryl isostearate, pentaglyceryl caprate, pentaglyceryl laurate, pentaglyceryl myristate, pentaglyceryl oleate, pentaglyceryl stearate, and pentaglyceryl isostearate, and more particularly from decaglyceryl caprate and decaglyceryl laurate.

16. Composition according to Claim 10 or 11, **characterized in that** the amount of methylenebis(hydroxyphenylbenzotriazole) compound of formula (IV) in the aqueous dispersion ranges from 10% to 60% by weight, and more preferentially from 30% to 50% by weight, relative to the total weight of the dispersion.

17. Composition according to any one of Claims 10 to 12, **characterized in that** the methylenebis(hydroxyphenylbenzotriazole) compound/mono-(C₈-C₂₀)alkyl ester of polyglycerol weight ratio ranges from 0.05 to 0.5, and more preferentially from 0.1 to 0.3.

18. Composition according to any one of Claims 9 to 13, **characterized in that** the methylenebis(hydroxyphenyl benzotriazole) compound of formula (IV) in the form of an aqueous dispersion is the 2,2'-methylenebis[6-(2H-benzotriazol-2-yl)-4-(1,1,3,3-tetramethylbutyl)phenol] compound having the structure below:

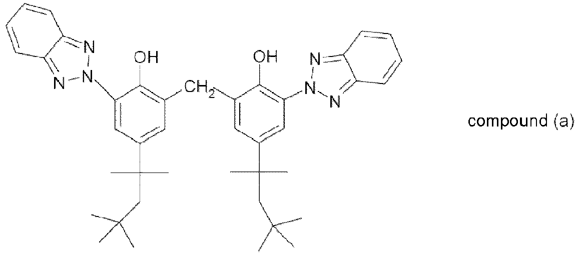

19. Composition according to any one of Claims 1 to 14, **characterized in that** the insoluble organic UV-screening agent(s) of the invention are present at a concentration of active material ranging from 0.1% to 15% by weight, and more particularly from 0.5% 10% by weight, relative to the total weight of the composition.

20. Composition according to any one of the preceding claims, **characterized in that** it also comprises at least one soluble organic UV-screening agent which is active in the UV-A range and/or the UV-B range, and/or an inorganic UV-screening agent.

21. Composition according to the preceding claim, **characterized in that** the soluble organic UV-screening agents are chosen in particular from anthranilates; cinnamic derivatives; dibenzoylmethane derivatives; salicylic derivatives; camphor derivatives; triazine derivatives; benzophenone derivatives; β,β'-diphenylacrylate derivatives; benzotriazole derivatives; benzalmalonate derivatives; benzimidazole derivatives; imidazolines; bis-benzazolyl derivatives; p-aminobenzoic acid (PABA) derivatives; screening polymers and screening silicones; α-alkylstyrene-based dimers; 4,4-diarylbutadienes and mixtures thereof.

22. Composition according to either of Claims 20 and 21, **characterized in that** the soluble organic UV-screening agents are chosen from:
- Ethylhexyl salicylate,
- Butylmethoxydibenzoylmethane,
- Ethylhexyl methoxycinnamate,
- Octocrylene,
- Phenylbenzimidazolesulfonic acid,
- Terephthalylidenedicamphorsulfonic acid,
- Benzophenone-3,
- Benzophenone-4,
- Benzophenone-5,
- 4-Methylbenzylidenecamphor,
- Benzimidazilate,
- Anisotriazine,
- Ethylhexyl triazone,
- Diethylhexyl butamidotriazone,
- Drometrizole trisiloxane,
and mixtures thereof.

23. Composition according to any one of the preceding claims, **characterized in that** it also comprises at least one adjuvant chosen from fatty substances, organic solvents, ionic or non-ionic thickeners, demulcents, antioxidants, free-radical scavengers, opacifiers, stabilizers, emollients, silicones, α-hydroxy acids, anti-foams, moisturizing agents, vitamins, insect repellents, fragrances, preservatives, surfactants, fillers, photoprotective agents, polymers other than those of the invention, propellants, basifying or acidifying agents, and dyes.

## Patentansprüche

1. Zusammensetzung, insbesondere Kosmetikzusammensetzung, umfassend mindestens ein photoprotektives System, das in der Lage ist, UV-Strahlen herauszufiltern, umfassend mindestens ein unlösliches organisches UV-Schutzmittel a), **dadurch gekennzeichnet, dass** es auch mindestens ein Polymer b) umfasst, das Monomereinheiten der Formeln (A) und (B) umfasst: 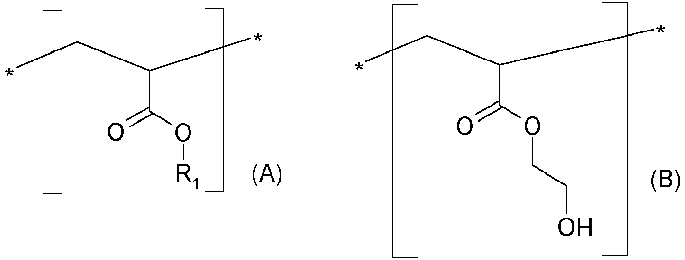 wobei:
- R₁, unabhängig voneinander, ausgewählt ist aus Alkyl- oder Alkylen-Radikalen,
- und
- mindestens 60 Gew.-% der R₁-Gruppen Behenyl-Radikale sind, wobei sich die Gewichtsprozent auf die Summe sämtlicher R₁-Gruppen beziehen, die in dem Polymer vorhanden sind,
- und
- das Gewichtsverhältnis der Summe sämtlicher Hydroxyethylacrylat-Einheiten zu der Summe sämtlicher Acrylat-Einheiten, die die R₁-Gruppe tragen, im Bereich von 1 : 30 bis 1 : 1 liegt,
- und die Summe der Gesamtheit der Einheiten A und B mindestens 95 Gew.-% des Gesamtgewichts des Polymers beträgt,
- das Polymer ein zahlenmittleres Molekulargewicht Mn im Bereich von 2000 bis 9000 g/mol aufweist.

2. Zusammensetzung nach Anspruch 1, **dadurch gekennzeichnet, dass** in dem Polymer b) R₁ aus Alkyl-Radikalen besteht, vorzugsweise aus C₁₆-C₂₂-Alkyl-Radikalen und besonders bevorzugt aus Behenyl(C₂₂)-Radikalen.

3. Zusammensetzung nach einem der Ansprüche 1 bis 2, **dadurch gekennzeichnet, dass** in dem Polymer b) mindestens 70 Gew.-% der R₁-Gruppen Behenyl-Radikale sind, vorzugsweise mindestens 80 Gew.-% und besonders bevorzugt mindestens 90 Gew.-%.

4. Zusammensetzung nach einem der Ansprüche 1 bis 3, **dadurch gekennzeichnet, dass** in dem Polymer b) sämtliche R₁-Gruppen Behenyl-Radikale sind.

5. Zusammensetzung nach einem der Ansprüche 1 bis 4, **dadurch gekennzeichnet, dass** in dem Polymer b) das Gewichtsverhältnis der Summe sämtlicher Hydroxyethylacrylat-Einheiten zu der Summe sämtlicher Acrylat-Einheiten, die die R₁-Gruppe tragen, im Bereich von 1 : 15 bis 1 : 1 liegt, vorzugsweise im Bereich von 1 : 10 bis 1 : 4, liegt.

6. Zusammensetzung nach einem der Ansprüche 1 bis 5, wobei die Polymereinheiten, die in dem Polymer b) vorhanden sind, aus den Einheiten (A) und (B) bestehen, die in einem der Ansprüche 1 bis 5 beschrieben sind.

7. Zusammensetzung nach einem der Ansprüche 1 bis 6, **dadurch gekennzeichnet, dass** das Polymer b) ein zahlenmittleres Molekulargewicht Mn im Bereich von 5000 bis 9000 g/mol aufweist.

8. Zusammensetzung nach einem der Ansprüche 1 bis 7, **dadurch gekennzeichnet, dass** das Polymer b) einen Schmelzpunkt im Bereich von 60 °C bis 69 °C und vorzugsweise im Bereich von 63 °C bis 67 °C aufweist.

9. Zusammensetzung nach einem der Ansprüche 1 bis 8, **dadurch gekennzeichnet, dass** das Polymer / die Polymere b) in der Zusammensetzung in einer Menge eines Aktivmaterials im Bereich von 0,1 Gew.-% bis 10 Gew.-%, vorzugsweise von 0,2 Gew.-% bis 4 Gew.-%, bezogen auf das Gesamtgewicht der Zusammensetzung, vorhanden sein kann/können.

10. Zusammensetzung nach einem der Ansprüche 1 bis 9, **dadurch gekennzeichnet, dass** das unlösliche organische UV-Schutzmittel in Form von Teilchen vorliegt, die eine mittlere Größe im Bereich von 0,01 bis 5 µm und besonders bevorzugt von 0,01 bis 2 µm und insbesondere von 0,020 bis 2 µm aufweisen.

11. Zusammensetzung nach einem der Ansprüche 1 bis 10, **dadurch gekennzeichnet, dass** das unlösliche organische UV-Schutzmittel insbesondere aus organischen UV-Schutzmitteln des Oxalanilid-Typs, des Triazin-Typs, des Benzotriazol-Typs; des Vinylamid-Typs; des Cinnamid-Typs; des Typs, der eine oder mehrere Gruppen umfasst, die Benzazol und/oder Benzofuran sind, Benzothiophen oder des Indol-Typs; des Aryl-Vinylen-Keton-Typs; des Phenylen-bis-benzoxazinon-Derivat-Typs; des Amid-, Sulfonamid- oder Acrylonitrilcarbamat-Derivat-Typs oder Gemischen davon ausgewählt ist.

12. Zusammensetzung nach einem der Ansprüche 1 bis 11, **dadurch gekennzeichnet, dass** das unlösliche organische UV-Schutzmittel ausgewählt ist aus:
(i) symmetrischen Triazinen, die durch Naphthalenyl-Gruppen oder Polyphenyl-Gruppen substituiert sind, insbesondere 2,4,6-Tris(diphenyl)triazin und/oder 2,4,6-Tris(ter-phenyl)triazin;
(ii) den Methylenbis(hydroxyphenylbenzotriazol)-Verbindungen der nachstehenden Formel (IV): 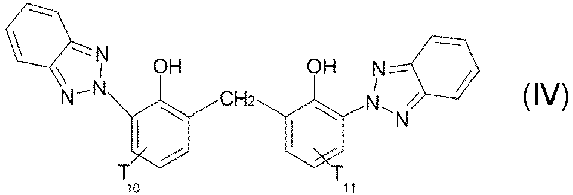 wobei die Radikale T₁₀ und T₁₁, die identisch oder unterschiedlich sein können, ein Ci-Cis-Alkyl-Radikal bezeichnen, das durch ein oder mehrere Radikale substituiert sein kann, die ausgewählt sind aus C₁-C₄-Alkyl, C₅-C₁₂-Cycloalkyl oder einem Arylrest;
(iii) und Gemischen davon.

13. Zusammensetzung nach einem der Ansprüche 12, **dadurch gekennzeichnet, dass** die Methylenbis(hydroxyphenylbenzotriazol)-Verbindung der Formel (IV) in Form einer wässrigen Dispersion von Teilchen mit einer mittleren Teilchengröße, die im Bereich von 0,01 bis 5 µm und besonders bevorzugt von 0,01 bis 2 µm und insbesondere von 0,020 bis 2 µm liegt, in Anwesenheit von mindestens einem Tensid mit der Struktur CₙH₂ₙ₊₁O (C₆H₁₀O₅)ₓH vorliegt, wobei n eine ganze Zahl von 8 bis 16 ist und x der mittlere Grad der Polymerisation der Einheit (C₆H₁₀O₅) ist und im Bereich von 1,4 bis 1,6 liegt.

14. Zusammensetzung nach Anspruch 11 oder 12, **dadurch gekennzeichnet, dass** die Methylenbis(hydroxyphenylbenzotriazol)-Verbindung der Formel (IV) in Form einer wässrigen Dispersion von Teilchen mit einer mittleren Teilchengröße, die im Bereich von 0,02 bis 2 µm und besonders bevorzugt von 0,01 bis 1,5 µm und insbesondere von 0,02 bis 1 µm liegt, in Anwesenheit von mindestens einem Mono(C₈-C₂₀)alkylester von Polyglycerin mit einem Grad der Glycerin-Polymerisation von mindestens 5 vorliegt.

15. Zusammensetzung nach einem der Ansprüche 14, **dadurch gekennzeichnet, dass** der Mono- (C₈-C₂₀) alkylester von Polyglycerin ausgewählt ist aus Decaglycerylcaprat, Decaglyceryllaurat, Decaglycerylmyristat, Decaglyceryloleat, Decaglycerylstearat, Decaglycerylisostearat, Hexaglycerylcaprat, Hexaglyceryllaurat, Hexaglycerylmyristat, Hexaglyceryloleat, Hexaglycerylstearat, Hexaglycerylisostearat, Pentaglycerylcaprat, Pentaglyceryllaurat, Pentaglycerylmyristat, Pentaglyceryloleat, Pentaglycerylstearat und Pentaglycerylisostearat, und insbesondere aus Decaglycerylcaprat und Decaglyceryllaurat.

16. Zusammensetzung nach einem der Ansprüche 10 oder 11, **dadurch gekennzeichnet, dass** die Menge an Methylenbis(hydroxyphenylbenzotriazol)-Verbindung der Formel (IV) in der wässrigen Dispersion im Bereich von 10 Gew.-% bis 60 Gew.-% und besonders bevorzugt von 30 Gew.-% bis 50 Gew.-%, bezogen auf das Gesamtgewicht der Dispersion, liegt.

17. Zusammensetzung nach einem der Ansprüche 10 bis 12, **dadurch gekennzeichnet, dass** das Gewichtsverhältnis von Methylenbis(hydroxyphenylbenzotriazol)-Verbindung zu Mono-(C₈-C₂₀) alkylester von Polyglycerin im Bereich von 0,05 bis 0,5 und besonders bevorzugt von 0,1 bis 0,3 liegt.

18. Zusammensetzung nach einem der Ansprüche 9 bis 13, **dadurch gekennzeichnet, dass** die Methylenbis(hydroxyphenylbenzotriazol)-Verbindung der Formel (IV) in Form einer wässrigen Dispersion die 2,2'-Methylenbis[6-(2H-benzotriazol-2-yl)-4-(1,1,3,3-tetramethylbutyl)phenol]-Verbindung mit der folgenden Struktur ist:

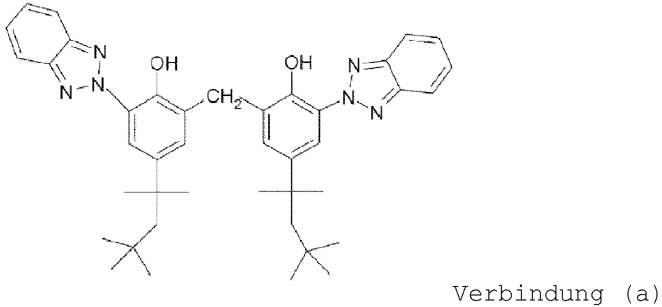

19. Zusammensetzung nach einem der Ansprüche 1 bis 14, **dadurch gekennzeichnet, dass** das/die unlösliche(n) organische(n) UV-Schutzmittel der Erfindung in einer Konzentration von Aktivmaterial im Bereich von 0,1 Gew.-% bis 15 Gew.-% und insbesondere von 0,5 % bis 10 Gew.-%, bezogen auf das Gesamtgewicht der Zusammensetzung, vorhanden ist/sind.

20. Zusammensetzung nach einem der vorstehenden Ansprüche, **dadurch gekennzeichnet, dass** sie auch mindestens ein lösliches organisches UV-Schutzmittel, das aktiv im UV-A-Bereich und/oder im UV-B-Bereich ist, und/oder ein anorganisches UV-Schutzmittel umfasst.

21. Zusammensetzung nach dem vorstehenden Anspruch, **dadurch gekennzeichnet, dass** die löslichen organischen UV-Schutzmittel insbesondere ausgewählt sind aus Anthranilaten; Zimtsäure-Derivaten; Dibenzoylmethan-Derivaten; Salicylsäure-Derivaten; Kampfer-Derivaten; Triazin-Derivaten; Benzophenon-Derivaten; β,β'-Diphenylacrylat-Derivaten; Benzotriazol-Derivaten; Benzalmalonat-Derivaten; Benzimidazol-Derivaten; Imidazolinen; Bisbenzazolyl-Derivaten; p-Aminobenzoesäure(PABA)-Derivaten; schützenden Polymeren und schützenden Silikonen; auf α-Alkylstyrol basierenden Dimeren; 4,4-Diarylbutadienen und Gemischen davon.

22. Zusammensetzung nach einem der Ansprüche 20 und 21, **dadurch gekennzeichnet, dass** die löslichen organischen UV-Schutzmittel ausgewählt sind aus:
- Ethylhexylsalicylat,
- Butylmethoxydibenzoylmethan,
- Ethylhexylmethoxycinnamat,
- Octocrylen,
- Phenylbenzimidazolsulfonsäure,
- Terephthalylidendikampfersulfonsäure,
- Benzophenon-3,
- Benzophenon-4,
- Benzophenon-5,
- 4-Methylbenzylidenkampfer,
- Benzimidazilat,
- Anisotriazin,
- Ethylhexyltriazon,
- Diethylhexylbutamidotriazon,
- Drometrizoltrisiloxan
und Gemischen davon.

23. Zusammensetzung nach einem der vorstehenden Ansprüche, **dadurch gekennzeichnet, dass** sie auch mindestens ein Adjuvans umfasst, das ausgewählt ist aus Fettsäurestoffen, organischen Lösungsmitteln, ionischen oder nichtionischen Verdickungsmitteln, Demulzenzien, Antioxidanzien, Radikalfängern, Trübungsmitteln, Stabilisatoren, Weichmachern, Silikonen, α-Hydroxysäuren, Antischäumen, Feuchthaltemitteln, Vitaminen, Insektenschutzmitteln, Düften, Konservierungsmitteln, Tensiden, Füllstoffen, photoprotektiven Mitteln, anderen als den erfindungsgemäßen Polymeren, Treibmitteln, Alkalisierungs- oder Säuerungsmitteln und Farbstoffen.

## Revendications

1. Composition, en particulier composition cosmétique, comportant au moins un système photoprotecteur capable de filtrer des rayons UV comprenant au moins un agent écran d'UV organique insoluble a), **caractérisée en ce qu'**elle comprend en plus au moins un polymère b) comprenant des unités monomériques de formules (A) et (B) 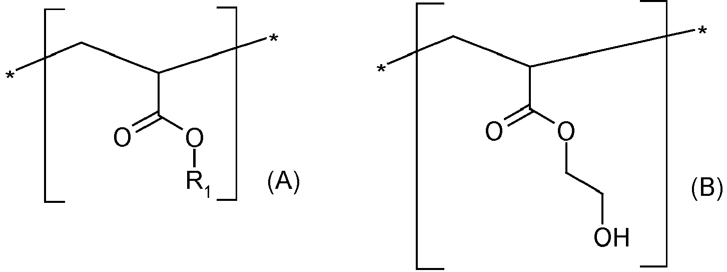 dans lesquelles :
- R₁, indépendamment l'un de l'autre, est choisi parmi les radicaux alkyle ou alkylène,
- et
- au moins 60 % en poids des groupes R₁ sont des radicaux béhényle, le pourcentage en poids se rapportant à la somme de tous les groupes R₁ présents dans le polymère,
- et
- le ratio pondéral de la somme de toutes les unités acrylate d'hydroxyéthyle sur la somme de toutes les unités acrylate portant le groupe R₁ va de 1 : 30 à 1 : 1 ;
- et la somme du total des unités A et B est d'au moins 95 % en poids du poids total du polymère,
- le polymère ayant un poids moléculaire moyen en nombre Mn allant de 2 000 à 9 000 g/mole.

2. Composition selon la revendication 1, **caractérisée en ce que** dans le polymère b) R₁ est constitué de radicaux alkyle, de préférence de radicaux alkyle en C_{16-C22}, et plus préférentiellement de radicaux béhényle (en C₂₂).

3. Composition selon l'une ou l'autre des revendications 1 à 2, **caractérisée en ce que** dans le polymère b) au moins 70 % en poids des groupes R₁ sont des radicaux béhényle, préférentiellement au moins 80 % en poids, et plus préférentiellement au moins 90 % en poids.

4. Composition selon l'une quelconque des revendications 1 à 3, **caractérisée en ce que** dans le polymère b) tous les groupes R₁ sont des radicaux béhényle.

5. Composition selon l'une quelconque des revendications 1 à 4, **caractérisée en ce que** dans le polymère b) le ratio pondéral de la somme de toutes les unités acrylate d'hydroxyéthyle sur la somme de toutes les unités acrylate portant le groupe R₁ va de 1 : 15 à 1 : 1, préférentiellement va de 1 : 10 à 1 : 4.

6. Composition selon l'une quelconque des revendications 1 à 5, dans laquelle les unités polymériques présentes dans le polymère b) sont constituées des unités (A) et (B) décrites dans l'une quelconque des revendications 1 à 5.

7. Composition selon l'une quelconque des revendications 1 à 6, **caractérisée en ce que** le polymère b) a un poids moléculaire moyen en nombre Mn allant de 5 000 à 9000 g/mole.

8. Composition selon l'une quelconque des revendications 1 à 7, **caractérisée en ce que** le polymère b) a un point de fusion allant de 60 °C à 69 °C, et préférentiellement allant de 63 °C à 67 °C.

9. Composition selon l'une quelconque des revendications 1 à 8, **caractérisée en ce que** le ou les polymères b) peu(ven)t être présent(s) dans la composition en une quantité de matière active allant de 0,1 % à 10 % en poids, de préférence de 0,2 % à 4 % en poids, par rapport au poids total de la composition.

10. Composition selon l'une quelconque des revendications 1 à 9, **caractérisée en ce que** l'agent écran d'UV organique insoluble est sous la forme de particules de taille moyenne allant de 0,01 à 5 um et plus préférentiellement de 0,01 à 2 um et plus particulièrement de 0,020 à 2 µm.

11. Composition selon l'une quelconque des revendications 1 à 10, **caractérisée en ce que** l'agent écran d'UV organique insoluble est choisi parmi les agents écrans d'UV organiques du type oxalanilide, du type triazine, du type benzotriazole ; du type vinylamide ; du type cinnamide ; du type comportant un ou plusieurs groupes qui sont benzazole et/ou benzofuranne, benzothiophènene ou du type indole ; du type arylvinylènecétone ; du type dérivé de phénylène bis-benzoxazinone, du type dérivé d'amide, de sulfonamide ou de carbamate d'acrylonitrile ou leurs mélanges.

12. Composition selon l'une quelconque des revendications 1 à 11, **caractérisée en ce que** l'agent écran d'UV organique insoluble est choisi parmi :
(i) les triazines symétriques substituées par des groupes naphtalényles ou des groupes polyphényles, en particulier la 2,4,6-tris(di-phényl)-triazine et/ou la 2,4,6-tris(ter-phényl)-triazine ;
(ii) les composés méthylène bis-(hydroxyphénylbenzotriazole) de formule (IV) suivante : 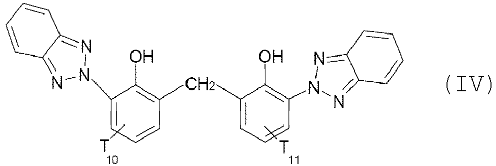 dans laquelle les radicaux T₁₀ et T₁₁, qui peuvent être identiques ou différents, désignent un radical alkyle en C₁-C₁₈ qui peut être substitué par un ou plusieurs radicaux choisis parmi alkyle en C₁-C₄, cycloalkyle en C₅-C₁₂ ou un radical aryle ;
(iii) et leurs mélanges.

13. Composition selon la revendications 12, **caractérisée en ce que** le composé méthylènebis-(hydroxyphénylbenzotriazole) de formule (IV) est sous la forme d'une dispersion aqueuse de particules ayant une taille moyenne de particule qui va de 0,01 à 5 um, et plus préférentiellement de 0,01 à 2 um, et plus particulièrement de 0,020 à 2 um, en la présence d'au moins un tensioactif de structure CₙH₂ₙ₊₁O(C₆H₁₀O₅)ₓH dans laquelle n est un entier de 8 à 16 et x est le degré moyen de polymérisation de l'unité (C₆H₁₀O₅) et va de 1,4 à 1, 6.

14. Composition selon la revendication 11 ou 12, **caractérisée en ce que** le composé méthylènebis-(hydroxyphénylbenzotriazole) de formule (IV) est sous la forme d'une dispersion aqueuse de particules ayant une taille moyenne de particule qui va de 0,02 à 2 um, et plus préférentiellement de 0,01 à 1,5 um, et plus particulièrement de 0,02 à 1 um, en la présence d'au moins un mono- (C₈-C₂₀)alkyl-ester de polyglycérol ayant un degré de polymérisation de glycérol d'au moins 5.

15. Composition selon la revendications 14, **caractérisée en ce que** le mono-(C₈-C₂₀)alkyl-ester de polyglycérol est choisi parmi le caprate de décaglycéryle, le laurate de décaglycéryle, le myristate de décaglycéryle, l'oléate de décaglycéryle, le stéarate de décaglycéryle, l'isostéarate de décaglycéryle, le caprate d'hexaglycéryle, le laurate d'hexaglycéryle, le myristate d'hexaglycéryle, l'oléate d'hexaglycéryle, le stéarate d'hexaglycéryle, l'isostéarate d'hexaglycéryle, le caprate de pentaglycéryle, le laurate de pentaglycéryle, le myristate de pentaglycéryle, l'oléate de pentaglycéryle, le stéarate de pentaglycéryle, l'isostéarate de pentaglycéryle et plus particulièrement parmi le caprate de décaglycéryle et le laurate de décaglycéryle.

16. Composition selon la revendication 10 ou 11, **caractérisée en ce que** la quantité de composé méthylènebis(hydroxyphénylbenzotriazole) de formule (IV) dans la dispersion aqueuse va de 10 % à 60 % en poids, et plus préférentiellement de 30 % à 50 % en poids, par rapport au poids total de la dispersion.

17. Composition selon l'une quelconque des revendications 10 à 12, **caractérisée en ce que** le rapport en poids de composé méthylènebis-(hydroxyphénylbenzotriazole)/mono-(C₈-C₂₀) alkyl-ester de polyglycérol va de 0,05 à 0,5, et plus préférentiellement de 0,1 à 0,3.

18. Composition selon l'une quelconque des revendications 9 à 13, **caractérisée en ce que** le composé méthylènebis-(hydroxyphénylbenzotriazole) de formule (IV) sous forme d'une dispersion aqueuse est le composé 2,2'-méthylène-bis-[6-(2H-benzotriazol-2-yl)-4-(1,1,3,3-tetraméthylbutyl)phénol] ayant la structure suivante :

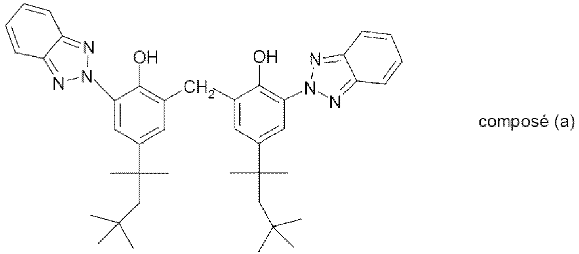

19. Composition selon l'une quelconque des revendications 1 à 14, **caractérisée en ce que** l'agent ou les agents écrans d'UV organiques insolubles de l'invention sont présents à une concentration en matière active allant de 0,1 % à 15 % en poids, et plus particulièrement de 0,5 % à 10 % en poids, par rapport au poids total de la composition.

20. Composition selon l'une quelconque des revendications précédentes, **caractérisée en ce qu'**elle comprend également au moins un agent écran d'UV organique soluble qui est actif dans la plage d'UV-A et/ou d'UV-B et/ou un agent écran d'UV inorganique.

21. Composition selon la revendication précédente, **caractérisée en ce que** les agents écrans d'UV organiques solubles sont choisis en particulier parmi les anthranilates ; les dérivés cinnamiques ; les dérivés de dibenzoylméthane ; les dérivés salicyliques ; les dérivés du camphre ; les dérivés de triazine ; les dérivés de la benzophénone ; les dérivés de β,β'-diphénylacrylate ; les dérivés de benzotriazole ; les dérivés de benzalmalonate ; les dérivés de benzimidazole ; les imadazolines ; les dérivés bis-benzoazolyle ; les dérivés de l'acide p-aminobenzoïque (PABA) ; les polymères filtrants et les silicones filtrantes ; les dimères dérivés d'a-alkylstyrène ; les 4,4-diaylbutadiènes et leurs mélanges.

22. Composition selon l'une ou l'autre des revendications 20 et 21, **caractérisée en ce que** les agents écrans d'UV organiques solubles sont choisis parmi :
- Ethylhexyl Salicylate,
- Butyl Methoxydibenzoylmethane,
- Ethylhexyl Methoxycinnamate,
- Octocrylene,
- Phenylbenzimidazolesulfonic Acid,
- Terephthalylidenedicamphorsulfonic Acid,
- Benzophenone-3,
- Benzophenone-4,
- Benzophenone-5,
- 4-Methylbenzylidenecamphor,
- Benzimidazilate,
- Anisotriazine,
- Ethylhexyl Triazone,
- Diethylhexyl Butamido Triazone,
- Drometrizole Trisiloxane,
et leurs mélanges.

23. Composition selon l'une quelconque des revendications précédentes, **caractérisée par le fait qu'**elle comprend également au moins un adjuvant choisi parmi les substances gras, les solvants organiques, les épaississants ioniques ou non ioniques, les émollients, les antioxydants, les agents de piégeage de radicaux libres, les agents opacifiants, les stabilisants, les émollients, les silicones, les α-hydroxyacides, les agents anti-mousse, les agents hydratants, les vitamines, les agents répulsifs contre les insectes, les fragrances, les conservateurs, les tensioactifs, les charges, les agents photoprotecteurs, les polymères autres que ceux de l'invention, les propulseurs, les agents alcalinisants ou acidifiants, et les colorants.
